# EUROPÄISCHE PATENTSCHRIFT

(11) **EP 1 563 963 B1**
(45) Veröffentlichungstag und Bekanntmachung des Hinweises auf die Patenterteilung: **20.09.2006**
(21) Anmeldenummer: 05003219.2
(22) Anmeldetag: 16.02.2005
(51) Int. Cl.: B25G 1/12

(54) **Schraubendreher mit einer Haltevorrichtung**
Screwdriver with holding device
Tournevis avec dispositif de maintien

(30) Priorität: 30.07.2004 DE 102004037429; 16.02.2004 DE 202004002440 U; 05.03.2004 DE 102004010794; 21.07.2004 DE 102004035384
(43) Veröffentlichungstag der Anmeldung: 17.08.2005
(73) Patentinhaber: FELO-Werkzeugfabrik Holland-Letz GmbH, 35279 Neustadt (DE)
(72) Erfinder: Holland-Letz, Martin, 35279 Neustadt (DE)
(74) Vertreter: Rehberg Hüppe + Partner

(56) Entgegenhaltungen:
- WO-A-01/14103
- DE-A1- 10 254 339
- DE-U1- 20 218 876
- DE-U1- 29 720 468
- US-A- 1 460 654
- US-A- 5 910 196
- US-A- 5 911 798
- US-A- 6 155 143
- US-A1- 2004 055 424
- US-B1- 6 199 872
- US-B1- 6 202 512
- US-B1- 6 223 633
- US-B1- 6 240 810
- US-B1- 6 393 949

## Beschreibung

Die Erfindung betrifft einen Schraubendreher mit einem Griff, einem auswechselbaren Funktionsteil mit einem Endstück und einer Haltevorrichtung, mittels welcher das Funktionsteil in einer gesicherten Stellung der Haltevorrichtung zumindest in eine axiale Richtung gegenüber dem Griff gesichert ist.

### STAND DER TECHNIK

Aus dem Stand der Technik sind grundsätzlich unterschiedliche Ausführungsformen von Schraubendrehern bekannt:
a) Schraubendreher mit nicht lösbar in einem Griff angeordneten Klingen.
b) Schraubendreher, bei denen in eine Höhlung eines Griffs unter formschlüssiger Aufnahme in Umfangsrichtung lösbar eine Schraubendreher-Klinge oder ein Funktionsteil einsetzbar ist, wodurch ein Betrieb desselben Griffs mit mehreren Schraubendreher-Klingen mit unterschiedlichen Funktionsspitzen möglich ist (s. a. DE 1 242 520). Auf diese Weise können Schraubendreher-Sätze geschaffen werden, die zumeist in einer Kassette zusammengefasst sind und unterschiedliche Funktionsteile mit verschiedenen Funktionsspitzen aufweisen. Die Funktionsspitzen sind passend für verschiedene Schraubengrößen und Schraubentypen ausgebildet, beispielsweise Schlitzschrauben, Kreuzschrauben oder Schrauben mit TORX®-Profil oder auch kleinere Steckschlüssel-Köpfe.
c) Insbesondere radial klein bauende Spezialschraubendreher, welche im Wesentlichen aus einer Klinge ohne einen Griff bestehen und im Endbereich über einen Drehteller verfügen, der von einem Benutzer axial beaufschlagt werden kann zur Anpressung der Schraubendreher-Klinge an eine Schraube.
d) Schraubendreher, die bis auf die Funktionsspitze der Schraubendreher-Klinge mit einem Isoliermantel, vorzugsweise aus Kunststoff, versehen sind zum Arbeiten an elektrischen Anlagen (vgl. DE-GM 7 135 254).
e) Schraubendreher, die einen metallischen Kern oder Schaft aufweisen, der in einem einem Griff gegenüberliegenden Endbereich eine Aufnahme für verschiedene Einsätze ("Bits") mit unterschiedlichen Funktionsspitzen bildet.

Aus der Druckschrift DE 44 01 335 A1 ist ein Schraubendreher bekannt, bei dem eine Klinge in einem Kern aufgenommen ist. Der Kern sitzt unlösbar kraftschlüssig in dem Griff des Schraubendrehers und weist zur Betätigungsmomentübertragung einen unrunden Querschnitt auf. Hierbei kann der Kern an dem klingenseitigen Ende axial geschlitzt sein, so dass der Kern nach der Art eines Dübels in die Höhlung des Griffs eingesetzt werden kann. Entsprechend einer weiteren Ausgestaltung dieser bekannten Ausführungsform kann die formschlüssige Befestigung des Kerns in der Aufnahme des Griffs als lösbare Schnappverbindung ausgebildet sein, um ein Handwerkzeug mit einem Griff und auswechselbarer Klinge zu schaffen. Weiterhin wird in der genannten Druckschrift eine elektrische Isolierung nach VDE-Bestimmungen angesprochen, für die der Kern um die Klinge gespritzt werden soll und die Klinge auch über den Griff hinaus bis in den vorderen Wirkbereich der Klinge überziehen soll. Hierdurch sollen Kriechstrombrücken zu der Klinge im Griffbereich ausgeschlossen werden. Der Griff ist dabei als Kunststoff-Spritzgussteil ausgebildet.

Aus DE-GM 453825 ist ein Taschen-Schraubendreher bekannt, dessen Klinge in einen Griff eingeschoben werden kann und in einer Betriebsstellung durch ein formschlüssig in eine Nut der Klinge eingreifendes Halteelement mit dem Griff verriegelbar ist. Die Verriegelung kann durch einen radial zu betätigenden Druckknopf gelöst werden.

Bei einem bekannten Schraubendrehersatz des Unternehmens Vessel Co., Thailand mit der Bezeichnung "FAMIDORA EIGHT" (No. TD 800, Barcode - Nr.: 4 907 587 061300) sind mehrere Funktionsteile mit unterschiedlichen Funktionsspitzen auswechselbar in einen einzigen Griff einsetzbar. Die Funktionsteile besitzen jeweils ein Endstück aus Kunststoff, welches in dem Bereich der Übertragung des Betätigungsmomentes einen konstanten Querschnitt aufweist und formschlüssig in Umfangsrichtung mit dem Griff in Wirkverbindung tritt. In der Betriebsstellung greift eine elastische Nase des Griffes in eine korrespondierende Nut des Endstückes ein. Werden Entnahmekräfte auf das Funktionsteil in axialer Richtung desselben ausgeübt, die ausreichend sind, um die Nase radial nach außen zu verformen, so kann das Funktionsteil aus dem Griff entfernt werden.

Aus dem Katalog des Unternehmens Wera "Wera - Der Schraubwerkzeugkatalog", Ausgabe 1993, Seiten 26-29 sowie 38-43, ist ein Schraubendreher bekannt, bei dem eine Klingenisolierung und der Griff selbst in einem Arbeitsgang nahtfrei und durchgehend gespritzt werden.

Weitere Ausführungsformen einer Verbindung einer Klinge oder eines Werkzeugs mit einem Griff oder einer Halterung sind beispielsweise aus den Druckschriften DE 102 19 418 A1, DE 197 07 839 A1, US 6,363,820 B1, US 2002/0170394 A1, US 1,473,186 und US 5,957,014 bekannt.

WO 01/14103 A offenbart einen Schraubendreher, bei dem ein Klingenschaft fest, nämlich stoffschlüssig, in einen Griff eingebunden ist. In den Klingenschaft ist auswechselbar ein Schraubendreher-Einsatz oder Bit einsetzbar. Der Klingenschaft ist einstückig im Spritzgussverfahren hergestellt.

US 5,911,798 A betrifft einen Drehmoment-Schlüssel, bei dem ein Funktionsteil als auf den Schlüssel aufsteckbare Nuss ausgebildet ist. Der Hebelarm des Drehmoment-Schlüssels ist teleskopierbar mit zwei Raststellungen für vordefinierte Hebelarm-Längen. In den Raststellungen tritt eine Nase eines Federelementes in eine Nut ein. Eine Lösung der Rastierung erfolgt durch Aufbringung einer Axialkraft auf den Drehmoment-Schlüssel, die infolge einer Abschrägung des Kontaktbereiches zwischen Nase und Nut zu einer elastischen Verformung der Nase quer zur Längsachse des Drehmoment-Schlüssels führt. Für hinreichende Axialkraft und hiermit korrespondierender hinreichender Bewegung der Nase kann dann die Länge des Drehmoment-Schlüssels verstellt werden.

US 1,460,654 offenbart eine als Blattfeder ausgebildete Haltevorrichtung für ein Werkzeug. Die Blattfeder ist an einem Schaft des Werkzeugs befestigt und weist einen Knopf auf, der in eine Durchbrechung im Schaft eingreift. Mit Hilfe eines Nagels soll der Knopf aus der Durchbrechung am Schaft/Griff des Werkzeugs herausgedrückt werden, um dann ein Herausziehen des Schafts zu ermöglichen.

Weiteren, teilweise gattungsfremden Stand der Technik sollen die Druckschriften DE 202 18 876 U1, US 6,393,949 B1, DE 102 54 339 A1 und DE 297 20 468 U1 darstellen.

### AUFGABE DER ERFINDUNG

Aufgabe der vorliegenden Erfindung ist es, einen Schraubendreher mit einem auswechselbaren Funktionsteil zu schaffen, der hinsichtlich
- einer Montage und Demontage von Funktionsteil und Griff,
- einer Ausrichtung von Griff und Funktionsteil,
- der Abstützung von Anpresskräften in Längsrichtung des Schraubendrehers,
- der Übertragung von Betätigungsmomenten und/oder
- der Isolierung des Schraubendrehers
verbessert ist.

### LÖSUNG

Die Aufgabe der Erfindung wird erfindungsgemäß mit den Merkmalen des unabhängigen Patentanspruchs 1 gelöst.

### BESCHREIBUNG DER ERFINDUNG

Die vorliegende Erfindung schlägt den Einsatz eines Schraubendrehers mit einem Griff vor, in den auswechselbar ein Funktionsteil eingesetzt werden kann. Durch eine lösbare Verbindung zwischen Funktionsteil und Griff sind grundsätzlich zwei unterschiedliche Betriebsarten möglich:
- Das Funktionsteil kann ohne Griff zum Anziehen einer Schraube eingesetzt werden. Bei einem derartigen Einsatz ist die Abmessung des Bereiches zum Halten und Drehen des Werkzeuges quer zu der Längserstreckung des Funktionsteiles verhältnismäßig klein infolge des fehlenden größeren Griffes, so dass das Werkzeug bspw. auch an nur durch kleine Öffnungen zugänglichen Montagestätten eingesetzt werden kann, beispielsweise für besonders feinfühlige Schraubarbeiten.
- Durch eine Verbindung mit einem Griff kann für eine Betätigung der Außendurchmesser vergrößert werden, wodurch eine Aufbringung vergrößerter Betätigungsmomente möglich ist. Die äußere Formgebung des Griffs kann zu einer verbesserten Haptik führen, wozu neben der Konturgestaltung des Griffs auch die Materialwahl des Griffs und die verbundenen mechanischen Eigenschaften beitragen können.

Durch die erfindungsgemäße Auswechselbarkeit des Funktionsteiles kann weiterhin derselbe Griff mit mehreren Funktionsteilen für unterschiedliche Befestigungselemente verwendet werden.

Erfindungsgemäß verfügt das Funktionsteil über ein Endstück, welches die Verbindung zwischen Funktionsteil und Griff unterstützt oder herbeiführt. Für den Fall, dass das Endstück aus einem anderen Material hergestellt ist als die Klinge oder ein Klingenschaft, kann die Bearbeitung und das Material für die beiden vorgenannten Bauteile separat gestaltet werden: Während beispielsweise für die Klinge ein Metall eingesetzt werden kann, welches entsprechend nachbehandelt sein kann zur Erzielung eines hochfesten Klingenschafts oder der Funktionsspitze des Klingenschafts, kann das Material des Endstücks hinsichtlich einer guten Auswechselbarkeit, eines geringen Gewichts, geeigneter Kontaktflächen mit dem Griff, der Isolationseigenschaften o. ä. ausgelegt werden.

Die Erfindung soll insbesondere ermöglichen, dass für den Monteur Sätze aus Funktionsteilen für eine Vielzahl von Schraubengrößen und -typen und einem Griff raum- und gewichtssparend in einer Kassette untergebracht werden, so dass der Monteur nicht eine entsprechende Zahl von vollständigen Schraubendrehern mit nicht auswechselbarem Funktionsteil, viel Gewicht und Raumbedarf mit sich führen muss.

Erfindungsgemäß ist weiterhin eine Haltevorrichtung vorgesehen. Die Haltevorrichtung weist ausgehend von einer Betriebsstellung, in der das Funktionsteil geeignet für eine Aufbringung eines Betätigungsmomentes in den Griff eingebracht ist, zwei unterschiedliche Stellungen auf:
- In einer *gesicherten Stellung* ist das Funktionsteil durch die Haltevorrichtung zumindest in eine axiale Richtung gegenüber dem Griff gesichert. In der gesicherten Stellung erfolgt damit der Betrieb des Schraubendrehers, wobei der Griff zur Übertragung eines Betätigungsmoments mit dem Funktionsteil verbunden ist.
- In einer *gelösten Stellung* kann das Funktionsteil relativ zum Griff bewegt werden, beispielsweise verdreht und/oder translatorisch bewegt werden.

Durch die Haltevorrichtung kann daher ein unbeabsichtigtes Lösen des Funktionsteils von dem Griff vermieden werden, was zu einem Herausfallen des Funktionsteils führen könnte. Weiterhin kann vermieden werden, dass sich das Funktionsteil unerwünscht selbsttätig aus der Betriebsstellung bewegt und Betätigungsmomente auf den Schraubendreher aufgebracht werden in einer Stellung des Funktionsteiles, in der eine Übertragung von Betätigungsmomenten nicht möglich ist, was zu Beschädigungen im Kontaktbereich zwischen Griff und Funktionsteil führen könnte.

Die Haltevorrichtung lässt sich durch manuelle Betätigung durch den Benutzer von der gesicherten Stellung der Haltevorrichtung in die gelöste Stellung verbringen. Hierzu weist die Haltevorrichtung beispielsweise ein Betätigungselement auf, welches durch einen Finger oder die Hand des Benutzers betätigt wird.

Weiterhin ist der erfindungsgemäße Schraubendreher für ein Arbeiten an unter elektrischer Spannung stehenden Anlagen angepasst, wozu das Funktionsteil teilweise oder mit Ausnahme der Funktionsspitze mit einem Isoliermantel und einem isolierenden Endstück ummantelt ist. Dieser Ausgestaltung liegt die Erkenntnis zugrunde, dass bekannte Schraubendreher mit auswechselbaren Funktionsteilen nicht für Arbeiten an unter elektrischer Spannung stehenden Anlagen geeignet sind, selbst wenn die Schäfte der Funktionsteile bis zur Funktionsspitze mit einem elektrisch isolierenden Mantel aus Kunststoff umhüllt sind. Um die beim Arbeiten auftretenden Betätigungsmomente sicher übertragen zu können, ist nämlich in den Griff aus Kunststoff oftmals ein Metallfutter verdrehfest eingesetzt, in das die Funktionsteile aus Stahl mit einem unrunden Schaftende formschlüssig eingesetzt werden. Dieses Schaftende muss auch bei solchen Funktionsteilen, die im Übrigen mit einem Isoliermantel aus Kunststoff umhüllt sind, ohne Ummantelung bleiben, weil eine Kunststoff-Ummantelung unter Betätigungsmomentbelastung aufreißen würde. Bei einer anderen bekannten Ausführungsform ist zwar in den Griff kein Metallfutter eingesetzt, sondern das Schaftende aus Stahl reicht sehr weit in den Griff hinein, um die ausreichend große Fläche zur Übertragung des Betätigungsmoments zu erhalten und die zusätzliche spezifische Druckbelastung des Kunststoffs, aus dem der Griff gefertigt ist, nicht zu überschreiten.

Nach der Norm EN 60900 müssen Werkzeuge, die zum Arbeiten an unter Spannung stehenden Anlagen geeignet sein sollen, mit den Griffen in einem Wasserbad eingetaucht gegen eine an der metallischen Funktionsspitze angelegte Spannung von 10.000 Volt zum Griff hin isoliert sein. Ein Schraubendreher, bei dem etwa das in den Griff eingesetzte Metallfutter von der Stirnseite des Griffs her tief in den Griff zurückgesetzt ist, bei dem das Funktionsteil bis auf das freie Schaftende isolierend ummantelt ist und mit dem ummantelten Bereich tief in die Höhlung des Griffs vor dem zurückgesetzten Futter eintaucht, würde dennoch nicht die geforderte Prüfung auf vollkommene Isolierung bestehen, weil das Wasser in die Höhlung eindringen würde. Hierdurch wird eine leitende Verbindung von der Funktionsspitze über das freie Schaftende zu dem in das Wasserbad eingetauchten Gegenpol der angelegten Prüfspannung hergestellt. Die nach EN 60900 für zusammensteckbare Werkzeuge auch zulässige Isolationsprüfung in einem Bad aus Stahlkugeln ist bei der geforderten 100%igen Stückprüfung bei großen Stückzahlen zu umständlich. Außerdem würde die Isolierung des Griffs gegenüber der Funktionsspitze bei einem zusammengesteckten Werkzeug nicht geleistet, wenn das Werkzeug etwa feuchter Witterung ausgesetzt ist und Wasser in den Spalt zwischen Ummantelung des Schafts und der Höhlung des Griffs eindringt.

Die genannten Effekte werden entsprechend der Erfindung dadurch vermieden, dass in dem Griff der Klingenschaft mit einem Isoliermantel und einem isolierenden Endstück ummantelt ist, so dass eine Übertragung einer elektrischen Spannung zwischen Klingenschaft und Griff und damit zu dem Benutzer des Schraubendrehers ausgeschlossen ist.

Besondere Aufmerksamkeit kommt hierbei der Haltevorrichtung zu, da vermieden werden muss, dass eine elektrische Brücke zwischen Griff und Klingenschaft durch die Haltevorrichtung geschaffen ist. Hier hat die Erfindung erkannt, dass es völlig ausreichend ist, wenn die Haltevorrichtung nicht auf den Klingenschaft oder eine Mitnehmerfläche einwirkt, also mit einem Bauteil aus Metall, sondern vielmehr mit dem Isoliermantel und/oder dem Endstück zusammenwirkt, also auf eine Mantelfläche von Isoliermantel oder Endstück einwirkt, die beispielsweise aus Kunststoff hergestellt sind. Hierbei ist es denkbar, dass Isoliermantel und/oder Endstück mit mehreren Materialien, verschiedenen Kunststoffen oder einem Verbundwerkstoff gebildet sind, derart, dass ein erstes innen liegendes Material für eine Isolierung verantwortlich ist, während ein auf der Mantelfläche angeordnetes Material geeignet an ein Zusammenwirken mit der Haltevorrichtung angepasst ist. Alternativ ist es ebenfalls denkbar, dass zwar die Haltevorrichtung auf einen nicht isolierten Bereich des Funktionsteiles einwirkt, aber selber beispielsweise mit Kunststoff oder einer Isolierung gebildet ist, so dass eine elektrische Brücke zwischen Benutzer und Klinge vermieden ist.

Die Konstruktion der Haltevorrichtung und die Auswahl der Materialien (insbesondere Kunststoffe) für Haltevorrichtung, Isoliermantel und/oder Endstück erfolgt so, dass die Forderungen der Norm 60900 an zusammengesetzte Werkzeuge erfüllt wird:

Demgemäß darf die Gebrauchsfähigkeit des Schraubendrehers im Temperaturbereich von - 20 °C bis + 70 °C nicht beeinträchtigt werden. Weiterhin müssen zusammengesetzte Schraubendreher Haltevorrichtungen gegen ein unbeabsichtigtes Lösen der Einzelteile haben. Diesbezüglich liegt der Erfindung der Gedanke zugrunde, dass eine Rast- oder Schnappverbindung nicht ausreichend ist. Bei tiefen Einsatztemperaturen können bei einem Lösen oder Einstecken des auswechselbaren Funktionsteiles in der Schnappverbindung Spannungen auftreten, die zu Rissen führen, welche die Isolationseigenschaften verschlechtern und die Betriebssicherheit des Schraubendrehers beeinträchtigen. Eine weitere Anforderung der Norm ist, dass Werkzeuge mit einer formschlüssigen Verbindung mit einer Kraft von 500 N auf ein unbeabsichtigtes Lösen der Einzelteile geprüft werden müssen. Eine derartige Prüfung für Schraubendreher mit einer Schnappverbindung birgt das Risiko in sich, dass diese bei hohen oder niedrigen Temperaturen versagen.

Nach einem weiteren Vorschlag der Erfindung wird das Betätigungselement der Haltevorrichtung von dem Benutzer in radialer Richtung des Griffs bewegt, um einen Wechsel von der gesicherten Stellung in die gelöste Stellung herbeizuführen. Dieser Ausgestaltung liegt die Erkenntnis zugrunde, dass eine radiale Betätigung des Betätigungselements einerseits, beispielsweise durch einen Daumen, bei in der Hand angeordnetem Griff für den Benutzer einfach und angenehm ist. Wenn die Hand des Benutzers den Griff des Schraubendrehers großflächig umgreift, ist für ein Aufbringen eines Betätigungsmoments auf den Schraubendreher eine unbeabsichtigte Betätigung des Betätigungselements in radialer Richtung (in einem ausreichenden Ausmaß für ein unbeabsichtigtes Verbringen der Haltevorrichtung in die gelöste Stellung) unwahrscheinlich, so dass ein unbeabsichtigtes Lösen des Funktionsteils von dem Griff ausgeschlossen ist. Dieser Effekt kann durch die Auswahl einer geeigneten Lage der Haltevorrichtung, beispielsweise am vorderen Endbereich des Griffes, unterstützt werden.

Darüber hinaus beruht die Erfindung auf der Erkenntnis, dass eine radiale Bewegung des Betätigungselements besonders einfach in eine Halte- oder Koppelwirkung zwischen Griff, Haltevorrichtung und Funktionsteil umgesetzt werden kann:
- Die radiale Bewegung kann einerseits mit radialen Kontaktkräften zwischen Griff, Funktionsteil und Haltevorrichtung verbunden sein, welche zu einer reibschlüssigen Verbindung zwischen Griff und Funktionsteil führen können.
- Andererseits kann durch die radiale Bewegung ein Halteelement in radialer Richtung in eine geeignete Aussparung, Vertiefung, hinter einen Vorsprung o. ä. eintreten, wodurch eine formschlüssige Verbindung zwischen Griff und Funktionsteil geschaffen werden kann. Derartige formschlüssige Verbindungen haben den Vorteil, dass u. U. bei kleinen Betätigungskräften des Betätigungselements dennoch große Haltekräfte, beispielsweise in eine oder beide axiale Richtungen des Funktionsteils, aufgebracht werden können. Weiterhin kann die Haltevorrichtung trotz der hohen erzeugten Haltekräfte auf einfache Weise in die gelöste Stellung überführt werden, da hierzu nicht die Haltekräfte überwunden werden müssen, sondern lediglich die formschlüssige Verbindung durch Beseitigung des Eingreifens des Halteelements in die Ausnehmung gelöst werden muss.

Alternativ oder zusätzlich kann eine Bewegung des Betätigungselements in Umfangrichtung erfolgen, beispielsweise um eine Verriegelung herzustellen oder zu lösen.

Vorzugsweise besitzt der Schraubendreher ein Federelement, welches bei manueller Betätigung des Betätigungselements in der gelösten Stellung beaufschlagt ist. Entfällt eine Betätigungskraft des Benutzers, so führt das Federelement das Betätigungselement und die Haltevorrichtung von der gelösten Stellung in die gesicherte Stellung zurück. Damit wird die Bedienung des Schraubendrehers im Zusammenhang mit der Auswechslung eines Funktionsteils erleichtert, da der Benutzer lediglich für ein Lösen auf das Betätigungselement einwirken muss. Gleichzeitig trägt das Federelement dafür Sorge, dass ohne äußere Kräfte auf das Betätigungselement zwingend die gesicherte Stellung der Haltevorrichtung eingenommen wird.

Nach einem weiteren Vorschlag der Erfindung weist die Haltevorrichtung ein Halteelement auf, welches mit dem Betätigungselement in Wirkverbindung steht. Das Halteelement kann beispielsweise einstückig mit dem Betätigungselement oder mit diesen über eine Antriebsverbindung, eine getriebliche Verbindung oder einen Hebel verbunden sein.

Bei einer bevorzugten Weiterbildung des Schraubendrehers weist die Haltevorrichtung einen Bügel auf. Der Bügel besitzt ein Unterteil, welches insbesondere das Halteelement bildet. Des Weiteren ist der Bügel über ein Seitenteil um die Mantelfläche des Funktionsteils herumgeführt. In dem dem Funktionsteil oder Endstück radial gegenüberliegenden Bereich steht der Bügel mit dem Betätigungselement in Wirkverbindung. Durch den erfindungsgemäßen Einsatz des Bügels, der um das Funktionsteil herumgeführt ist, kann erreicht werden, dass mit einer radialen Bewegung des Betätigungselements nach innen, also einer Bewegung des Betätigungselements in Richtung des Funktionsteils, das Halteelement von dem Funktionsteil weg bewegt wird. Infolge einer derartigen "Umkehr der Bewegungsrichtungen" durch den Bügel ist es auf einfache Weise ermöglicht, dass mit einem Betätigen des Betätigungselements radial nach innen ein Lösen des Funktionsteils von dem Griff erfolgt.

Die Bedienung des Schraubendrehers im Zusammenhang mit einer Auswechslung des Funktionsteils kann dadurch vereinfacht werden, dass während eines Einschiebens des Funktionsteils in den Griff die Haltevorrichtung automatisch in die gelöste Stellung gebracht wird. Dies kann beispielsweise dadurch erfolgen, dass das Halteelement an der Mantelfläche des Funktionsteils anliegt und, beispielsweise durch konusförmige Bereiche der Mantelfläche, mit einem Einschieben des Funktionsteils in den Griff von der gesicherten Stellung in Richtung der gelösten Stellung bewegt wird. Ist die Endstellung des Funktionsteils in dem Griff erreicht, so veranlasst das Federelement die Bewegung der Haltevorrichtung in die gesicherte Stellung, in der das Halteelement form- oder reibschlüssig in Wirkverbindung mit dem Funktionsteil tritt. Demgemäß muss für das Einschieben des Funktionsteils das Betätigungselement nicht betätigt werden - vielmehr können die Hände des Benutzers einerseits zum Halten des Griffs und andererseits zum Halten des Funktionsteils verwendet werden.

Eine besonders einfach und kostengünstig herzustellende Führung des Bügels bei gleichzeitig hoher Funktionalität ergibt sich, wenn dieser in radial orientierten Nuten des Griffs geführt ist, die von einer Durchbrechung auf der Oberseite des Griffs ausgehen, wobei in der Durchbrechung das Betätigungselement angeordnet ist.

Das von dem Halteelement beaufschlagte Federelement kann als separate Feder, beispielsweise als Druckfeder aus Federstahl, ausgebildet sein. Eine besonders einfache, aber funktionale Ausgestaltung ergibt sich allerdings, wenn das Federelement einstückig mit dem Bügel gebildet ist. Ist der Bügel als Kunststoffteil ausgebildet, so kann das Federelement als Fortsatz oder Nase oder Federzunge des Bügels ausgebildet sein. Ein solches Federelement bietet den Vorteil, dass es nicht extra montiert werden muss. Außerdem kann ein derartiges integrales Federelement aus Kunststoff nicht durch eine eindringende Feuchtigkeit korrodieren wie bspw. eine Stahlfeder.

Während eine Übertragung des Betätigungsmoments um die Längsachse des Schraubendrehers auch über eine reibschlüssige Verbindung in Umfangsrichtung möglich ist, ergibt sich eine besonders gute, steife und dauerhafte Übertragung des Betätigungsmoments, wenn in Umfangsrichtung eine formschlüssige Verbindung zwischen dem Griff und dem Endstück vorgesehen ist. Diese kann beispielsweise in einer unrunden, aber korrespondierenden Gestaltung der Außen- und Innenkonturen von Griff und Endstück bestehen. Weiterhin kann der Griff oder das Endstück über mindestens einen geeigneten radialen Vorsprung verfügen, welcher in eine entsprechende radiale Vertiefung des anderen genannten Bauteils eingreift.

Die Beanspruchung der Kontaktflächen kann dadurch vermindert werden, dass das Endstück im Bereich der Verbindung mit dem Griff vergrößerte Außenabmessungen aufweist, da hierdurch der Wirkdurchmesser vergrößert wird. Auf diese Weise wird das maximal übertragbare Betätigungsmoment vergrößert. Trotz eines Betriebes mit u. U. großen Betätigungsmomenten kann erfindungsgemäß der Einsatz von Kunststoff im Kontaktbereich zwischen Griff und Funktionsteil, welcher zu einer Isolation des Schraubendrehers führen kann, erst ermöglicht werden.

Gemäß einem weiteren Vorschlag der Erfindung ist der Bügel als eine Rahmenstruktur gebildet mit einem Unterteil, Seitenteilen und einem Oberteil. Eine derartige Rahmenstruktur stellt eine hinsichtlich der mechanischen Beanspruchungen verbesserte Struktur dar, die steif ist und eine hohe Sicherheit gegenüber einem Versagen aufweist. Gleichzeitig können die Seitenteile für eine Führung des Bügels, beispielsweise in den zuvor genannten Nuten, eingesetzt werden sowie zusätzlich einer Führung der Betätigungskräfte um das Funktionsteil herum dienen. Dabei kann die Aufteilung der Betätigungskräfte auf zwei Seitenteile zu einer verringerten Dimensionierung der Seitenteile führen, wodurch ein besonders kompakter Aufbau ermöglicht ist.

Weiterhin ermöglicht die Erfindung, dass zwischen Griff und Funktionsteil ein weiteres Federelement vorgesehen ist, welches in Richtung der Längsachse des Schraubendrehers, insbesondere mit dem Einschieben des Funktionsteils in den Griff, beaufschlagt wird und bei manueller Überführung der Haltevorrichtung in die gelöste Stellung das Funktionsteil zumindest teilweise aus dem Griff ausschiebt oder auswirft. Hierdurch ist ermöglicht, dass das Funktionsteil aus der Betriebsstellung nicht durch Zugkräfte entnommen werden muss, die auf das Funktionsteil vom Benutzer aufgebracht werden müssen. Vielmehr reicht es für ein Ausschieben oder Auswerfen des Funktionsteils aus, dass mit einem Finger das Betätigungselement betätigt wird. Das Ausschieben oder Auswerfen wird somit auf einfache Weise automatisiert - ein Auswerfen des Funktionsteiles bei Bedienung mit nur einer Hand im Bereich des Griffes ist ebenfalls möglich. Durch ein teilweise ausgeschobenes Funktionsteil kann auch für den Benutzer signalisiert werden, dass das Funktionsteil aus der Betriebsstellung gelöst ist und u. U. die Haltevorrichtung in der gelösten Stellung ist, so dass das Funktionsteil mit wenig Kraftaufwand aus dem Griff herausgezogen werden kann. Das Federelement kann bspw. eine Druckfeder aus Stahldraht sein, aber auch ein Zylinder aus elastischem Kunststoff.

Bei einer Weiterbildung des erfindungsgemäßen Schraubendrehers ist zusätzlich zu der Betriebsstellung eine Entnahmestellung vorgesehen, für die das Funktionsteil teilweise von dem Griff gelöst ist, jedoch gegenüber einem Herausfallen gesichert ist. In der Entnahmestellung kann das Funktionsteil von dem Benutzer aus dem Griff entnommen werden. Beispielsweise wird das Funktionsteil durch das weitere Federelement von der Betriebsstellung in die Entnahmestellung verbracht, in welcher das Funktionsteil noch durch eine elastische Rastvorrichtung gesichert ist. Durch Überwindung der Rastierkräfte der Rastvorrichtung durch den Benutzer kann das Funktionsteil entnommen werden. In diesem Fall erfolgt sowohl die Bewegung von der Betriebsstellung in die Entnahmestellung als auch die weitere Entnahme des Funktionsteils in Längsrichtung des Griffs. Alternativ ist es denkbar, dass eine Bewegung von der Betriebsstellung in die Entnahmestellung durch eine Verdrehung des Funktionsteils gegenüber dem Griff erfolgt, während die vollständige Entnahme des Funktionsteils durch eine Bewegung desselben in Längsrichtung des Griffs erfolgt.

Vorzugsweise ist das Funktionsteil als selbständiger Schraubendreher einsetzbar, bei dem der Klingenschaft mit dem Isoliermantel und/oder dem Endstück isoliert ist. Auf diese Weise ist der eingangs angeführte multifunktionale Einsatz des Funktionsteiles mit und ohne Griff auch an elektrischen Anlagen möglich.

Bei einer Weiterbildung des Schraubendrehers ist das Endstück in Längsrichtung mit einer Funktionsfläche konisch ausgebildet und in einer Längsrichtung an einer konischen Gegenfläche des Griffes abgestützt sowie in die andere Längsrichtung durch eine Haltevorrichtung gehalten.

Der Erfindung liegt zunächst die Erkenntnis zugrunde, dass für ein Sichern gegenüber einem Herausfallen des Funktionsteiles in eine Längsrichtung nur verhältnismäßig geringe Haltekräfte zwischen Griff und Funktionsteil erforderlich sind. Hingegen sind zur Übertragung der Drehmomente in die entgegengesetzte Längsrichtung des Schraubendrehers große Haltekräfte erforderlich. Eine Aufbringung derart großer Haltekräfte allein mittels üblicher Halte- oder Rastvorrichtungen ist nicht oder nur unter großem konstruktiven Aufwand möglich.

Erfindungsgemäß schlägt die Erfindung vor, die Haltekräfte in Entnahmerichtung durch die Haltevorrichtung aufzubringen. In die entgegengesetzte Richtung wird die Haltekraft (zumindest teilweise) durch die konusförmige Kontaktfläche des Endstückes aufgebracht, die an einer korrespondierenden innenkonusförmigen Fläche des Griffes zur Anlage kommt. Die konusförmigen Kontaktflächen bieten mehrere Vorteile:
- Die konusförmigen Anlageflächen erzeugen automatisch eine Zentrierwirkung, die eine schnelle und Fehler vermeidende Montage des Funktionsteiles in dem Griff unterstützt.
- Gleichzeitig ist in der Endstellung eine exakte radiale Ausrichtung des Funktionsteiles gegenüber dem Griff gewährleistet.
- Im Fall gleicher Konuswinkel der konusförmigen Flächen von Funktionsteil und Griff wird durch diese Flächen weiterhin die Orientierung des Funktionsteiles gegenüber dem Griff vorgegeben, so dass ein Fluchten der Längsachsen der vorgenannten Bauteile gewährleistet ist.
- Weiterhin wird je nach Wahl der Steigerung der konusförmigen Flächen eine gewisse Klemmwirkung erzeugt, so dass mit einer Vergrößerung der Anpressung des Schraubendrehers an eine Schraube sowohl die axiale Abstützkraft als auch radiale Führungskräfte vergrößert werden. Damit wird mit zunehmender Belastung des Schraubendrehers in axialer Richtung die Anbindung zwischen Funktionsteil und Griff verstärkt.

In Folge der Erkenntnis, dass zur Sicherung gegenüber einem Herausfallen des Funktionsteiles aus dem Griff nur eine kleinere Haltekraft in eine Richtung erforderlich ist, kann die Haltevorrichtung gezielt
- an eine geeignete Betätigung,
- geeignete Betätigungskräfte,
- einen günstigen Betätigungsort und
- die gewünschte Sicherungskraft
angepasst werden kann. Hierbei kann die Haltevorrichtung insbesondere konstruktiv einfacherer ausgebildet werden und/oder mit einem verringerten Bauraum ausgebildet werden.

Eine Entnahme des Funktionsteiles aus dem Griff kann vereinfacht werden, wenn die Konuswinkel größer gewählt werden als die Selbsthemmungswinkel der Materialpaarung, die für den Griff und das Endstück verwendet wird.

Gemäß einem weiteren Vorschlag der Erfindung stützt sich das auswechselbare Funktionsteil mit dem Endstück in einer Betriebsstellung mit einer ersten Funktionsfläche in eine axiale Richtung gegenüber einer korrespondierenden ersten Gegenfläche des Griffes ab. Hierbei handelt es sich beispielsweise um die vorgenannten Konusflächen oder quer zur Längsachse des Schraubendrehers orientierte Flächen. Der Griff weist ein Gewinde auf. Die Haltevorrichtung, beispielsweise eine "Überwurfmutter", besitzt eine zweite Gegenfläche und ein Gewinde. Die zweite Gegenfläche tritt mit einer zweiten Funktionsfläche des Funktionsteiles in Wechselwirkung. Die Haltevorrichtung und der Griff sind hierbei über eine Verschraubung der Gewinde miteinander verbunden. Über eine Veränderung der Verschraubung ist eine Veränderung des Abstandes der ersten Gegenfläche von der zweiten Gegenfläche und/oder eine Veränderung der Anpresskraft des Funktionsteiles an die erste Funktionsfläche und die zweite Funktionsfläche herbeiführbar. Über die Verschraubung wird das Funktionsstück zwischen den Gegenflächen "eingespannt". Die Verschraubung stellt einerseits eine besonders zuverlässige Haltevorrichtung bereit, welche auch unabhängig von etwaigen Toleranzen oder Fertigungsungenauigkeiten oder Temperaturdehnungen wirksam wird. Andererseits kann die Haltewirkung durch eine derartige Verschraubung gut eingestellt und ggf. angepasst werden.

Eine verbesserte Kraftentfaltung kann erzielt werden, wenn die zweite Gegenfläche von einer konusförmigen Innenfläche der Haltevorrichtung gebildet ist.

Vorteilhafte Weiterbildungen der Erfindung ergeben sich aus abhängigen Patentansprüchen und der gesamten Beschreibung. Weitere Merkmale sind den Zeichnungen - insbesondere den dargestellten Geometrien und den relativen Abmessungen mehrerer Bauteile zueinander sowie deren relativer Anordnung und Wirkverbindung - zu entnehmen. Die Kombination von Merkmalen unterschiedlicher Ausführungsformen der Erfindung oder von Merkmalen unterschiedlicher Patentansprüche abweichend von den gewählten Rückbeziehungen ist ebenfalls möglich und wird hiermit angeregt. Dies betrifft auch solche Merkmale, die in separaten Zeichnungsfiguren dargestellt sind oder bei deren Beschreibung genannt werden. Diese Merkmale können auch mit Merkmalen unterschiedlicher Patentansprüche kombiniert werden.

### KURZBESCHREIBUNG DER FIGUREN

Im Folgenden wird die Erfindung anhand in den Figuren dargestellter bevorzugter Ausführungsbeispiele weiter erläutert und beschrieben.
- **Fig. 1A**: zeigt einen Schraubendreher mit einem Griff und einem in den Griff eingesetzten Funktionsteil, wobei der Griff teilweise in einem Längsschnitt dargestellt ist.
- **Fig. 1B**: zeigt den Schraubendreher gemäß Fig. 1A mit einem anderen in den Griff eingesetzten Funktionsteil in Seitenansicht.
- **Fig.1C**: zeigt den Schraubendreher gemäß Fig. 1A mit einem weiteren in den Griff eingesetzten Funktionsteil in Seitenansicht.
- **Fig. 2**: zeigt ein mit einer Klinge und einer einstückigen Isolierung mit einem Isoliermantel und einem Endstück gebildetes Funktionsteil im Längsschnitt.
- **Fig. 2A**: zeigt ein mit einer Klinge und einer mehrstückigen Isolierung mit einem Isoliermantel und einem Endstück gebildetes Funktionsteil im Längsschnitt.
- **Fig. 3A**: zeigt einen Querschnitt III-III des Funktionsteils gemäß Fig. 2 oder 2A mit in erster Näherung rechteckigem Außenquerschnitt des Endstücks.
- **Fig. 3B**: zeigt einen Querschnitt III-III des Funktionsteils gemäß Fig. 2 oder 2A mit in erster Näherung Bogenstern-förmigem Außenquerschnitt des Endstücks.
- **Fig. 4**: zeigt ein rahmenförmiges Bauelement einer Haltevorrichtung mit einem Halteelement und einem Betätigungselement, wobei die Zeichenebene mit einer Querebene des Schraubendrehers korrespondiert.
- **Fig. 4A**: zeigt eine alternative Ausgestaltung des Bauelements der Haltevorrichtung mit integriertem Federelement, wobei die Zeichenebene mit einer Querebene des Schraubendrehers korrespondiert.
- **Fig. 5**: zeigt einen Querschnitt des Schraubendrehers bei Schnittführung V-V gemäß Fig. 6 für eine montierte Haltevorrichtung in einer gesicherten Stellung derselben, wobei das Funktionsteil in einer Betriebsstellung in den Griff eingesetzt ist.
- **Fig. 6**: zeigt einen Teillängsschnitt eines Schraubendrehers mit Haltevorrichtung in einer gesicherten Stellung, wobei das Funktionsteil in einer Betriebsstellung in den Griff eingesetzt ist.
- **Fig. 7**: zeigt eine zweite Ausgestaltung eines Schraubendrehers im Teillängsschnitt, wobei das Funktionsteil in eine axiale Richtung durch eine mit einer Schraubkappe ausgebildeten Haltevorrichtung gesichert ist.
- **Fig. 8**: zeigt eine dritte Ausgestaltung eines Schraubendrehers im Teillängsschnitt, wobei das Funktionsteil in eine axiale Richtung durch eine mit einer Schraubkappe, einer Konusfläche und einer zwischen Konusfläche und Funktionsteil ausgebildeten Haltevorrichtung gesichert ist.
- **Fig. 9**: zeigt eine vierte Ausgestaltung eines Schraubendrehers im Teillängsschnitt,
wobei das Funktionsteil durch eine Ringnut des Funktionsteils und einen elastischen Vorsprung der Haltevorrichtung gesichert ist.
- **Fig. 10**: zeigt eine fünfte Ausgestaltung eines Schraubendrehers in Teil-Seitenansicht, bei dem die Haltevorrichtung mit einer sattel- oder zungenförmigen Feder ausgebildet ist, die sich gegenüber dem Griff abstützt.
- **Fig. 11**: zeigt den Schraubendreher gemäß Fig. 10 im Teillängsschnitt XI-XI.
- **Fig. 12**: zeigt eine sechste Ausgestaltung eines Schraubendrehers im Teillängsschnitt, bei dem eine Haltevorrichtung mit einem eine reibschlüssige Verbindung gewährleistenden elastischen Ring gebildet ist.
- **Fig. 13**: zeigt eine siebte Ausgestaltung eines Schraubendrehers im Teillängsschnitt, bei dem eine Verzahnung zwischen Endstück und Griff eine Übertragung von Betätigungsmomenten bei gleichzeitiger Ermöglichung eines axialen Freiheitsgrads gewährleistet.
- **Fig. 14**: zeigt eine achte Ausgestaltung eines Schraubendrehers im Teillängsschnitt, bei dem ein Betätigungsmoment reibschlüssig übertragen wird.
- **Fig. 15**: zeigt eine neunte Ausgestaltung eines Schraubendrehers im Teillängsschnitt, bei dem eine Übertragung von Betätigungsmomenten reibschlüssig erfolgt, wobei ein Gewindeansatz des Griffs durch eine Schraubkappe radial nach innen gegen eine Mantelfläche des Funktionsteils verspannt wird.
- **Fig. 16**: zeigt den Schraubendreher gemäß Fig. 15 im Querschnitt XVI-XVI.
- **Fig. 17**: zeigt ein Funktionsteil eines Schraubendrehers für einen Betrieb ohne Griff, wobei das Funktionsteil in einem Endbereich lösbar mit einem Drehkopf verbunden ist.
- **Fig. 18**: zeigt eine zehnte Ausgestaltung eines Schraubendrehers im Teillängsschnitt, bei dem das Endstück nur im Bereich eines Absatzes konusförmig ausgebildet ist und eine Einspannung des Endstückes über eine Schraubkappe erfolgt.
- **Fig. 19**: zeigt den Schraubendreher gemäß Fig. 18 im Querschnitt IXX-IXX.
- **Fig. 20**: zeigt eine elfte Ausgestaltung eines Schraubendrehers im Teillängsschnitt mit einer Haltevorrichtung, die durch eine Verdrehung einer Drehkappe um die Längsachse des Schraubendrehers von einer gelösten Stellung in eine gesicherte Stellung überführt werden kann.
- **Fig. 21**: zeigt den Schraubendreher gemäß Fig. 20 im Querschnitt XXI-IXXI, wobei sich die Haltevorrichtung in einer gesicherten Stellung befindet.
- **Fig. 22**: zeigt den Schraubendreher gemäß Fig. 20 im Querschnitt XXI-IXXI, wobei sich die Haltevorrichtung in einer gesicherten Stellung befindet.
- **Fig. 23**: zeigt den Schraubendreher gemäß Fig. 20 im Querschnitt XXIII-IXXIII mit Anschlagnocken zur Begrenzung der Verdrehung der Haltevorrichtung.

### FIGURENBESCHREIBUNG

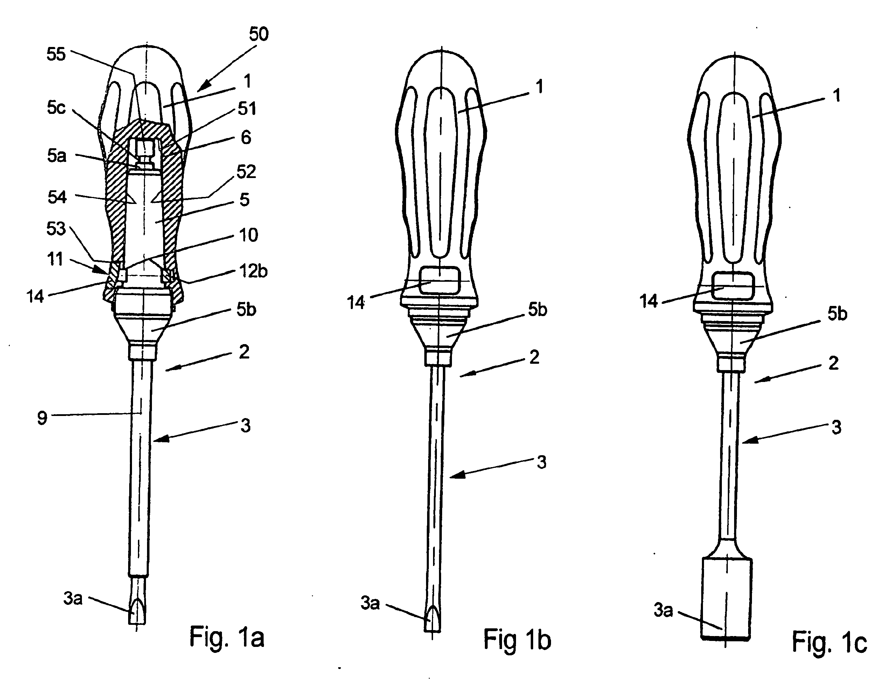

Gemäß **Fig. 1a** ist bei einem Schraubendreher 50 in einem Griff 1 ein Funktionsteil 2 derart angeordnet, dass ein von Hand auf den Griff 1 aufgebrachtes Betätigungsmoment auf das Funktionsteil 2 übertragen wird. Weiterhin wird eine in Längsrichtung auf den Griff 1 aufgebrachte Kraft auf einen Klingenschaft 3 und eine Funktionsspitze 3a des Funktionsteils 2 übertragen.

Der Griff besitzt eine Höhlung oder Ausnehmung 6, die
- von einer Stirnseite des Griffs 1 in Längsrichtung desselben ausgeht,
- einen Grund 51,
- mit einem Winkel α gegenüber der Längsachse geneigte Gegenflächen 52, die seitliche Begrenzungen der Höhlung 6 bilden, und
- eine radiale Durchbrechung 53
aufweist.

Das Funktionsteil 2 besitzt in der in den Fig. 1 a), b), c) dargestellten Betriebsstellung im Übergangsbereich zu dem Griff 1 einen Kopf 5b mit gegenüber dem übrigen Funktionsteil 2 vergrößertem Querschnitt, welcher im Wesentlichen dicht, mit enger Passung oder Übergangspassung radial in den zugeordneten Bereich des Griffs 1 eingepasst ist. Zur Abdichtung kann zwischen Kopf 5b und Griff 1 eine Dichtung, beispielsweise eine auf die Mantelfläche des Kopfes aufgebrachte elastische Schicht aus Kunststoff, zwischengeschaltet sein. An den Kopf 5b schließen sich in Richtung des Inneren des Griffs 1 unter dem Winkel α gegenüber der Längsachse geneigte Funktionsflächen 54 an. In dem innen liegenden Endbereich besitzt das Funktionsteil 2 einen Zapfen 5a, eine Ringnut 5c sowie einen Zapfenbund 55, welche in der genannten Reihenfolge in Längsrichtung hintereinander liegend angeordnet sind.

In der in den Figuren dargestellten Betriebsstellung ist das Funktionsteil 2 soweit in dem Griff eingeschoben, dass die Funktionsflächen 54 zur vollflächigen Anlage an den Gegenflächen 52 kommen und/oder die Stirnfläche des Zapfenbunds 55 zur Anlage an den Grund 51 kommen. Hierdurch ist die axiale Position des Funktionsteils 2 gegenüber dem Griff 1 in der Betriebsstellung vorgegeben. Über eine in die Durchbrechung 53 eingesetzte Haltevorrichtung 11 erfolgt eine zusätzliche Sicherung der axialen Position des Funktionsteils 2 und/oder eine Sicherung einer Verdrehung des Funktionsteils 2 gegenüber dem Griff 1. Insbesondere ist über die Haltevorrichtung 11 das Funktionsteil 2 gegenüber einer Bewegung aus dem Griff 1 gesichert. Die Funktionsflächen 54 und geeignete Bereiche für ein Zusammenwirken mit der Haltevorrichtung 11 werden von einem Endstück 5 des Funktionsteils 2 gebildet, welches in den Griff 1 hineinragt.

Das Endstück 5 des Funktionsteils 2 liegt mit den Funktionsflächen 54 an den Gegenflächen 52 der Höhlung 6 des Griffs 1 im Wesentlichen spielfrei an. Die Tiefe der Höhlung 6 ist etwas größer als die Länge des Endstücks 5. Der spielfreie Sitz ist durch die konische Form von Endstück 5 und Höhlung 6 gewährleistet. Der Konuswinkel α ist hierbei vorzugsweise größer gewählt als der Selbsthemmungswinkel der Materialpaarung der Materialien für den Griff 1 und das Endstück 5. Auf diese Weise wird vermieden, dass das Endstück 5 in der Höhlung 6 zu einem so festen Sitz kommt, dass das Funktionsteil 2 nicht mehr ohne Hilfsmittel aus dem Griff 1 herausgezogen werden kann. Bei einer alternativen Ausführung der Form- und Maßgestaltung von Endstück 5 und Höhlung 6 ist der Konuswinkel α kleiner als der Selbsthemmungswinkel. Allerdings sind die Weite und die Länge der Höhlung 6, die Außenform des Endstücks 5 und seine Länge mit dem Zapfen 5a und dem Zapfenbund 55 so abgestimmt, dass kein zu festes Verkeilen des Endstücks 5 in der Höhlung 6 erfolgt, weil der Zapfen 5a am Grund 51 der Höhlung 6 aufsitzt, dennoch ein weitgehend spielfreier Sitz erzielt wird.

Das Auftreten einer "Doppelpassung" dadurch, dass sowohl die Funktionsflächen 54 als auch der Zapfen 5a die Betriebsstellung des Funktionsteiles 2 in dem Griff vorgeben, kann vermieden werden, wenn die Funktionsflächen 54, die Gegenfläche 52 und/oder Zapfen 5a oder Zapfenbund 55 elastisch sind.

**Fig. 1A bis 1C** zeigen einen identischen Griff 1 mit drei unterschiedlichen Funktionsteilen 2, nämlich gemäß Fig. 1A ein Funktionsteil 3 mit einem Isoliermantel 9 und einer freien, bspw. für eine Schlitz-Schraube ausgebildeten Funktionsspitze 3a, gemäß Fig. 1B ein Funktionsteil 2 ohne Isoliermantel 9 mit einer für eine Schlitz-Schraube ausgebildeten Funktionsspitze sowie gemäß Fig. 1C ein Funktionsteil 2 mit einer vergrößerten Funktionsspitze 3a, beispielsweise für die Aufnahme eines Bits.

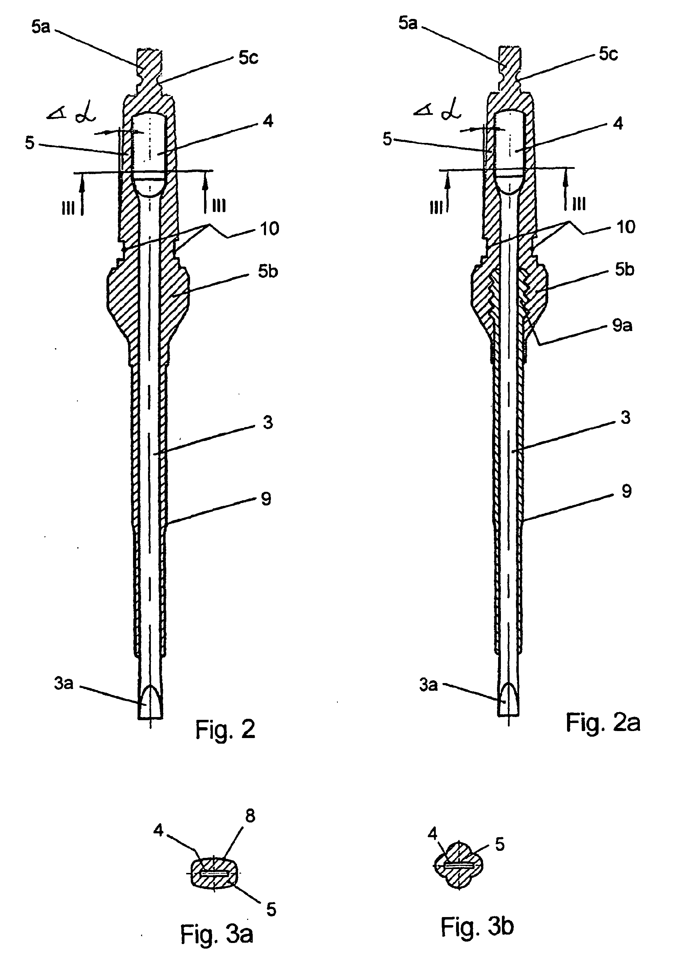

**Fig. 2** zeigt das Funktionsteil 2 mit dem einen Kern bildenden Klingenschaft 3 aus Metall, der in einem Endbereich die Funktionsspitze 3a bildet und in dem anderen Endbereich die Mitnehmerfläche 4 bildet, die gegenüber dem Klingenschaft eine zumindest in eine Querrichtung vergrößerte Dimension aufweist. Für die in den Fig. 2 und 3 dargestellten Ausführungsbeispiele ist der Klingenschaft 3 zylinderförmig ausgebildet, während die Mitnehmerfläche 4 eine Abplattung darstellt. Der Körper des Endstücks 5 ist zusammen mit dem Isoliermantel 9 des Klingenschafts 3 in einem Spritzgießvorgang auf den Klingenschaft 3 und die Mitnehmerfläche 4 aufgebracht. Endstück 5 und Isoliermantel 9 sind einstückig ausgebildet und umschließen den Klingenschaft 3 allseits bis auf die Funktionsspitze 3a und bilden eine elektrische Isolierung insbesondere zwischen Klingenschaft 3 und Griff 1.

Gemäß **Fig. 2A** sind Isoliermantel 9 und Endstück 5 nicht einstückig in einem Spritzgießvorgang auf den Klingenschaft 3 aufgebracht, sondern nacheinander gefertigt. In einem ersten Arbeitsgang wird der Klingenschaft 3 mit dem Isoliermantel 9 versehen, vorzugsweise in einem Spritzgießverfahren. In dem dem Griff 1 zugewandten Endbereich 9a weist der Isoliermantel 9 mehrere ringförmige prismatische Erhebungen auf, die konzentrisch zur Längsachse angeordnet sind. In einem zweiten Arbeitsgang wird das Endstück 5 aufgespritzt, dessen Kopf 5b den Endbereich 9a des Isoliermantels 9 dichtend umschließt.

In **Fig. 3A** ist die Lage der Mitnehmerfläche 4 zur seitlichen Erstreckung des Endstücks 5 zu erkennen. Die Mitnehmerfläche 4 liegt mit ihrer größten Breite in Richtung der größten seitlichen Erstreckung des Endstücks 5, was zur Folge hat, dass eine große Wandstärke der Kunststoffumhüllung auch an den Schmalseiten der Mitnehmerfläche 4 erzielt wird. Andererseits folgt hieraus ein großer Hebel bei der Übertragung eines Betätigungsmoments vom Griff 1 über das Endstück 5 auf die Mitnehmerfläche 4. In Fig. 3A ist zu erkennen, dass die Mitnehmerfläche 4 einen in erster Näherung rechteckförmigen Querschnitt aufweist und ebenfalls die Mantelfläche des Endstücks 5 im Querschnitt ungefähr rechteckförmig ist, wobei die Eckbereiche abgerundet sind und die Seitenflächen 8 des Endstücks in dem dargestellten Querschnitt konvex gewölbt sind.

**Fig. 3B** zeigt eine weitere Ausgestaltung eines möglichen Querschnitts des Endstücks 5, hier einen Bogenstern-förmigen Querschnitt. Die Innenkontur der Höhlung 6 ist korrespondierend zur Außenkontur des Endstücks 5 ausgebildet, so dass sich für eine Übertragung eines Betätigungsmoments um die Längsachse des Funktionsteils eine formschlüssige Übertragung zwischen Funktionsteil 2 und Griff 1 ergibt.

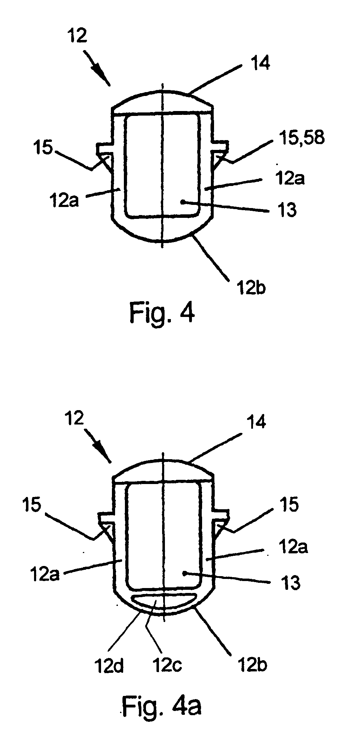

**Fig. 4** zeigt einen ring- oder rahmenförmig ausgebildeten Bügel 12 mit einem Betätigungselement oder Kopf 14, Seitenteilen 12a, einem Unterteil 12b und einer mittigen Durchbrechung 13, so dass ein ungefähr rechteckförmiger Rahmen gebildet ist. Der Betätigungskopf 14 ist abgerundet, und zwar ungefähr entsprechend der Außenkontur des Griffs 1. Das Unterteil 12b ist ebenfalls außenliegend abgerundet, nämlich entsprechend der Innenkontur des Grunds 57 der Durchbrechung 53. Der Bügel 12 weist Stütznocken 15 auf, die sich quer zu den Seitenteilen 12a auf beiden Seiten von diesen nach außen erstrecken. Die Stütznocken 15 sind ungefähr bei einem Drittel der Längserstreckung der Seitenteile 12a angeordnet. Auf der dem Betätigungskopf 14 gegenüberliegenden Seite gehen die Stütznocken 15 über Einführschrägen 58 in die Seitenteile 12a über.

Für die alternative Ausführungsform gemäß **Fig. 4A** ist das Unterteil 12b des Bügels 12 stärker ausgewölbt und weist eine Durchbrechung 12c auf, so dass ein brückenartiger Steg 12d gebildet ist. Wird der Steg 12d in Richtung der Durchbrechung 12c beaufschlagt, so verformt sich dieser, und es entsteht eine entgegengesetzte, im Einbauzustand radial wirkende Federkraft.

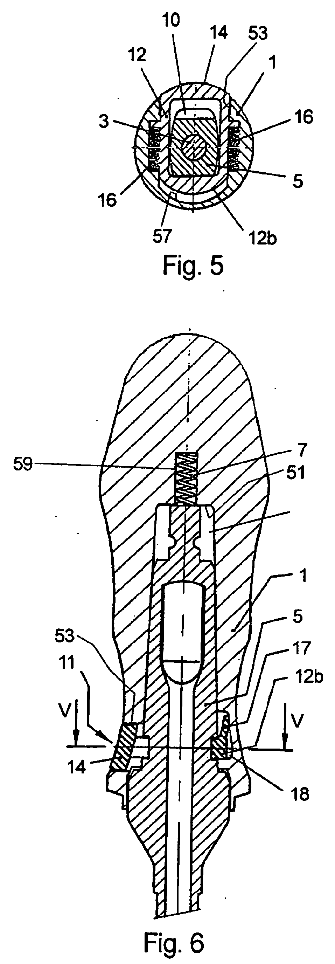

**Fig. 5** zeigt den Bügel 12, nachdem dieser in radialer Richtung in die Durchbrechung 53 des Griffs eingesetzt worden ist. Durch Druckfedern 16, die sich in einem Endbereich an dem Griff 1 abstützen und in dem gegenüberliegenden Endbereich an den Stütznocken 15 abstützen, ist der Bügel 12 und damit die Haltevorrichtung 11 in die in Fig. 5 dargestellte gesicherte Stellung beaufschlagt, in der der Betätigungskopf 14 bündig zur Außenkontur des Griffs ist. Manuell kann der Betätigungskopf 14 radial nach innen beaufschlagt werden, was mit einer Verschiebung des Bügels 12 radial nach innen und mit einer Beaufschlagung der Druckfedern 16 einhergeht, bis das Unterteil 12b zur Anlage an den Grund 57 des Griffs kommt. Der letztgenannte Zustand entspricht einer gelösten Stellung der Haltevorrichtung 11.

In der Betriebsstellung und gesicherten Stellung ist das Unterteil 12b des Bügels 12 formschlüssig in eine Ausnehmung 10 aus dem Endstück 5 eingetreten, wobei diese formschlüssige Verbindung durch die Druckfedern 16 und/oder den Steg 12d unterstützt oder aufrecht erhalten wird.

Mit einem Einsetzen des Bügels 12 in den Griff 1 werden die Seitenteile 12a in Richtung der Ausnehmung 13 eingebogen, so dass sich der Bügel 12 trotz der nach außen hervorstehenden Stütznocken 15 in die Durchbrechung 53 des Griffs 1 schieben lässt. Sobald die Stütznocken 15 in der Höhlung 6 im Griff 1 angeordnet sind, verformen sich die Seitenteile 12a wieder elastisch nach außen. Die Oberseiten der Stütznocken 15 bilden dann Anschläge, mit denen sie in der Betriebsstellung von unten an der Wandung der Höhlung 6 anliegen und verhindern, dass der Bügel durch die Kraft der Druckfedern 16 oder des Stegs 12d aus dem Griff 1 herausgedrückt wird.

Für die Ausführungsform gemäß Fig. 4A wird das Maß von der Oberseite der Stütznocken 15 bis zum tiefsten Punkt des Stegs 12d größer gewählt als der Abstand von der Innenwand der Höhlung 6 des Griffs 1 an der Stelle, an der die Stütznocken 15 mit ihrer Oberseite anliegen, bis zum tiefsten Punkt der Höhlung, in radialer Richtung gemessen. Infolgedessen ist der Steg 12d bei dem in den Griff eingesetzten Bügel 12 in Richtung der Durchbrechung 12c eingebogen und drückt federnd das Unterteil 12b des Bügels von unten in die Ausnehmung 10 im Endstück 5. Durch Druck auf den Betätigungskopf 14 wird der Bügel 12 weiter radial verschoben und der Steg 12d noch etwas weiter eingebogen. Die Höhe der Ausnehmung 12c und der Querschnitt des Stegs 12d sind so gewählt, dass die maximale Einbiegung ohne plastische Verformung möglich ist und die gewünschte Federkraft erzielt wird.

Gemäß Fig. 5 greift der Bügel 12 der Haltevorrichtung 11 in die untere Ausnehmung 10 im Endstück 5 ein, arretiert das Endstück 5 und dadurch das Funktionsteil 2 gegen ein unbeabsichtigtes Herausfallen aus dem Griff 1. Wird der Bügel 12 durch manuelle Betätigung des Betätigungskopfes 14 gegen die Federn 16 nach unten gedrückt, so wird das Unterteil 12b des Bügels 12 aus der unteren Ausnehmung 10 im Endstück 5 herausgedrückt, und das Funktionsteil 2 mit dem Endstück 5 kann aus dem Griff 1 herausgezogen werden. Ein anderes Funktionsteil kann in dieser Stellung des Bügels 12 in die Höhlung 6 des Griffs 1 eingesteckt werden. Wird kein Druck mehr auf den Bügel 12 ausgeübt, hebt sich der Bügel 12 infolge der Druckfeder 16 oder anderer Federelemente wieder und taucht in die Ausnehmung 10 im Endstück 5 ein, so dass dieses gesperrt ist.

Gemäß einer Weiterbildung der Erfindung ist es ebenfalls möglich, dass mit einem Einschieben des Funktionsteils 2 in den Griff 1 durch Kontaktflächen zwischen Funktionsteil 2, Endstück 5 und/oder Funktionsfläche 54 und Bügel 12, insbesondere Unterteil 12b, mit zunehmendem Einschieben der Bügel 12 automatisch von der gesicherten Stellung in Richtung der gelösten Stellung bewegt wird, bis mit einem Erreichen der Betriebsstellung das Unterteil 12 in die Ausnehmung 10 des Endstücks "einschnappt". Gemäß dieser Ausgestaltung muss für ein Einführen des Funktionsteils 2 in den Griff 1 die Haltevorrichtung 11 nicht zusätzlich manuell betätigt werden. Vielmehr muss der Betätigungskopf 14 lediglich in radialer Richtung betätigt werden, wenn das Funktionsteil 2 aus dem Griff 1 entnommen werden soll.

**Fig. 6** zeigt eine Ausführungsvariante des Bügels 12, an dessen Unterteil 12b ein zungenförmiger Ansatz 17 einstückig, beispielsweise aus einem elastischen Kunststoff, angeformt ist. Der Ansatz 17 erstreckt sich in Längsrichtung des Griffs 1 und ist gegen die Wandung der Höhlung 6 des Griffs 1 schräg geneigt. Die Winkelstellung und die Länge des Ansatzes 17 sind so gewählt, dass sich der Ansatz 17 federnd an der Wandung der Höhlung 6 abstützt und dadurch eine in radialer Richtung wirkende Kraft auf den Bügel 12 ausübt. Die Stärke der Kraft wird durch die Wahl des Querschnitts des Ansatzes 17 und seine Länge bestimmt. Der Ansatz 17 ist vorzugsweise in eine in Längsrichtung in die Wandung der Höhlung 6 eingeformte Vertiefung 18 eingelassen. In diesem Fall können die Druckfedern 16 entfallen oder zusätzlich eingesetzt werden.

Gemäß dem in Fig. 6 dargestellten Ausführungsbeispiel ist ausgehend von dem Grund 51 eine Bohrung 59 vorgesehen, in die eine Druckfeder 7 eingesetzt ist. Diese Druckfeder 7 ist in der Betriebsstellung von dem Funktionsteil 2 in Längsrichtung zusammengedrückt und vorgespannt. Wird durch Druck auf den Betätigungskopf 14 die Haltevorrichtung 11 in die gelöste Stellung gebracht, so wird die Arretierung des Endstücks 5 gelöst. Für Haltevorrichtung 11 in gelöster Stellung schiebt die Druckfeder 7 das Endstück 5 und damit das Funktionsteil 2 zumindest ein kleines Stück aus dem Griff 1 heraus.

Für die Haltevorrichtung 11 entsprechend den Fig. 1-6 tritt ein Halteelement, hier das Unterteil 12b, in radialer Richtung in Wirkverbindung mit einer Mantelfläche des Funktionsteils 2, insbesondere des Endstücks 5. Die gewünschte Haltewirkung kann hierbei durch eine reibschlüssige Verbindung hergestellt werden, welche sich infolge der zuvor erwähnten radialen Anpressung ergibt, und/oder durch eine formschlüssige Verbindung, beispielsweise eines radial beaufschlagten Elements wie das Halteelement, insbesondere Unterteil 12b, mit einer Nut oder Vertiefung in dem Funktionsteil 2. Die für die formschlüssige und/oder reibschlüssige Verbindung oder deren Aufrechterhaltung verantwortliche radiale Kraft infolge des Federelementes kann über eine manuelle Betätigung des Betätigungselements verändert oder beseitigt werden, womit eine Veränderung der Haltevorrichtung 11 von der gesicherten Stellung in die gelöste Stellung verbunden ist. Eine Bewegung von der gelösten Stellung in Richtung der gesicherten Stellung erfolgt durch geeignete, beispielsweise die dargestellten Federelemente.

Die Ausnehmung 10 und das Halteelement 12b sind für die formschlüssige Verbindung korrespondierend zueinander ausgebildet. Für die dargestellten Ausführungsformen bilden die Ausnehmung 10 und das Halteelement 12b in der gesicherten Stellung und der Betriebsstellung Kontaktflächen aus, die quer zur Längsachse des Schraubendrehers orientiert sind. Dies hat zur Folge, dass ohne eine manuelle Betätigung des Betätigungskopfes 14 das Funktionsteil 2 nicht bzw. nur bei einer Zerstörung des Halteelementes 12b aus dem Griff 1 herausgezogen werden kann. Für den Fall, dass abweichend zu den dargestellten Ausführungsformen die genannten Kontaktflächen mit einer Neigung gegenüber der Querebene ausgebildet sind, kann bei Überschreiten eine Zugkraft in Längsrichtung des Schraubendrehers 50 das Funktionsteil 2 auch ohne manuelle Betätigung des Betätigungskopfes 14 gelöst werden, wobei die erforderliche Zugkraft durch die Neigung, die Federkennlinie der Druckfedern 16 und die Reibparameter des die Ausnehmung 10 begrenzenden Materiales und des Halteelementes 12b konstruktiv vorgegeben werden kann.

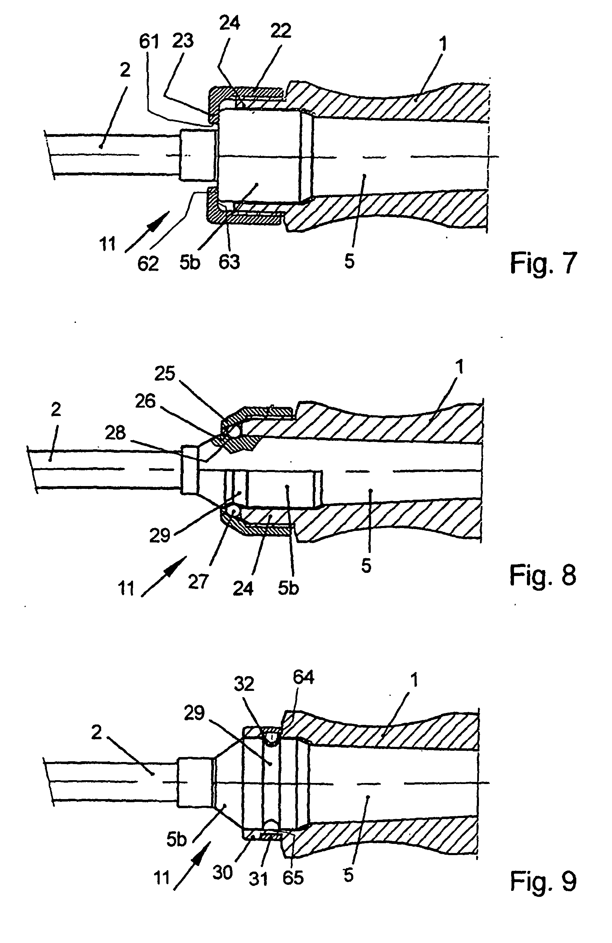

Eine alternative Haltewirkung ergibt sich aus der alternativen Ausführungsform gemäß **Fig. 7**. Bei ansonsten den voranstehend beschriebenen Ausführungsformen entsprechender Gestaltung der Abstützung in eine Längsrichtung sowie Übertragung des Betätigungsmoments ist gemäß Fig. 7 an der vorderen Seite des Griffs 1 einstückig ein zylindrischer Ansatz 24 mit Außengewinde angeformt. Auf den Ansatz 24 ist eine Schraubkappe 22 aus Kunststoff aufgesetzt und aufgeschraubt, die an ihrer Stirnwand 23 eine Bohrung 61 aufweist, deren Durchmesser kleiner ist als der Außendurchmesser des Kopfes 5b des Endstücks 5 oder der Stirnseite des Endstücks 5, wenn kein abgesetzter Kopf vorhanden ist. Zum Einsetzen des Endstücks 5 in den Griff 1 wird die Schraubkappe 22 vom Gewindeansatz 24 abgedreht, das Funktionsteil 2 mit dem Endstück 5 und Kopf 5b in die Höhlung 6 im Griff 1 eingesteckt, die Schraubkappe 22 über die Funktionsspitze 3a auf das Funktionsteil 2 aufgesteckt und auf den Gewindeansatz 24 aufgeschraubt. Die Stirnwand 23 der Schraubkappe 22 legt sich innen an die Stirnseite des Kopfes 5b an und presst das Endstück 5 in die Höhlung 6 des Griffs 1.

Die außen liegende Stirnfläche des Kopfes 5b bildet zusätzlich zu den Funktionsflächen 54 eine zweite Funktionsfläche 62, an welche mit zunehmender Verschraubung der Schraubkappe 22 die von der Stirnwand 23 gebildete zweite Gegenfläche 63 zur Anlage kommt. Mit zunehmender Verschraubung der Schraubkappe 22 mit dem Gewindeansatz 24 verringert sich somit der Abstand der Gegenflächen 63, 52 und eine Verspannung des Funktionsteils 2 zwischen den Gegenflächen 52, 63 kann vergrößert werden. Eine derartige Verspannung kann lediglich einer axialen Sicherung des Funktionsteils 2 dienen oder aber einer Erhöhung der Normalkraft zwischen Funktionsflächen 54, 62 sowie Gegenflächen 52, 63, so dass eine reibschlüssige Übertragung eines Betätigungsmoments verstärkt wird.

Gemäß der in **Fig. 8** dargestellten Ausführungsvariante der Haltevorrichtung 11 weist eine Schraubkappe 25 einen als zweite Gegenfläche 63 ausgebildeten Innenkonus 26 auf, über den eine Kugel 27 radial beaufschlagt wird, die in einer Radialbohrung im Gewindeeinsatz 24 geführt ist und deren Durchmesser größer ist als die Wandstärke des Gewindeansatzes 24. Wird die Schraubkappe 25 angezogen, so presst die zweite Gegenfläche 63 die Kugel 27 radial auf den Kopf 5b des Endstücks 5 und hält diesen durch Reibschluss fest. Eine in den Kopf 5b eingeformte Ringnut 28 kann eine zusätzliche formschlüssige Verbindung schaffen. Der Vorteil dieser Ausführungsvariante ist, dass die Schraubkappe 25 nicht vollständig abgeschraubt werden muss, um das Funktionsteil 2 mit dem Endstück 5 in den Griff 1 einzustecken, da die Bohrung in der Stirnwand der Schraubkappe 25 so groß sein kann, dass sich das Endstück 5 mit seinem Kopf 5b hindurchstecken lässt. Die Kugel 27 ist zweckmäßigerweise aus einem hochfesten Kunststoff hergestellt. Alternativ kann das Endstück 5 ohne Kopf 5b ausgebildet sein und im Vorderteil eine kalottenartige Vertiefung 28 aufweisen, in die die Kugel 27 eingepresst wird. Fig. 8 zeigt in der oberen Hälfte der Darstellung eine Ausführung, bei der das Endstück 5 ohne einen Kopf 5b ausgebildet ist und die Kugel 27 in einer kalottenartigen Vertiefung 28 im Endstück 5 sitzt. Die untere Hälfte der Darstellung zeigt eine Ausführung, bei der das Endstück 5 im vorderen Bereich einen Kopf 5b mit der Ringnut 29 aufweist, in der die Kugel 27 sitzt, wenn die Schraubkappe 25 zum Festhalten des Endstücks 5 angezogen wird. Wird die Schraubkappe 25 losgeschraubt, kann die Kugel 27 bei beiden Ausführungen radial ausweichen. Das Endstück 5 mit dem Funktionsteil 2 kann dann aus dem Griff 1 herausgezogen werden.

Bei einer weiteren Ausführungsform gemäß **Fig. 9** weist der Griff 1 an der Stirnseite einen Ansatz 30 auf, in dessen Umfangsfläche eine Ringnut 64 eingeformt ist. Von dieser Ringnut 64 geht an mindestens einer Stelle eine Durchbrechung 65 aus, die in die Bohrung in dem Ansatz 30 oder in die Höhlung 6 im Griff 1 führt. In die Ringnut 64 ist eine Ringfeder 31 eingesetzt. Die Ringfeder 31 ist vorzugsweise aus Kunststoff im Spritzgießverfahren hergestellt und besitzt einen kalottenförmigen Ansatz 32. Der kalottenförmige Ansatz 32 ragt durch die Durchbrechung 65 in die Bohrung oder in die Höhlung 6 im Griff 1 im Ansatz 30 hinein. In den Kopf 5b des Endstücks 5 oder in das Endstück 5 ohne einen Kopf 5b ist eine Ringnut 29 eingeformt, in die der kalottenförmige Ansatz 32 federnd einrastet, wenn das Funktionsteil 2 in den Griff 1 eingesteckt wird. Durch diese form- und kraftschlüssige Verbindung wird das Funktionsteil 2 im Griff 1 gehalten. Wird die Ringfeder 31 ausreichend stark ausgelegt, reicht zum Festhalten des Funktionsteils 2 im Griff 1 auch eine kraftschlüssige Verbindung, so dass die Ringnut 29 auch entfallen kann.

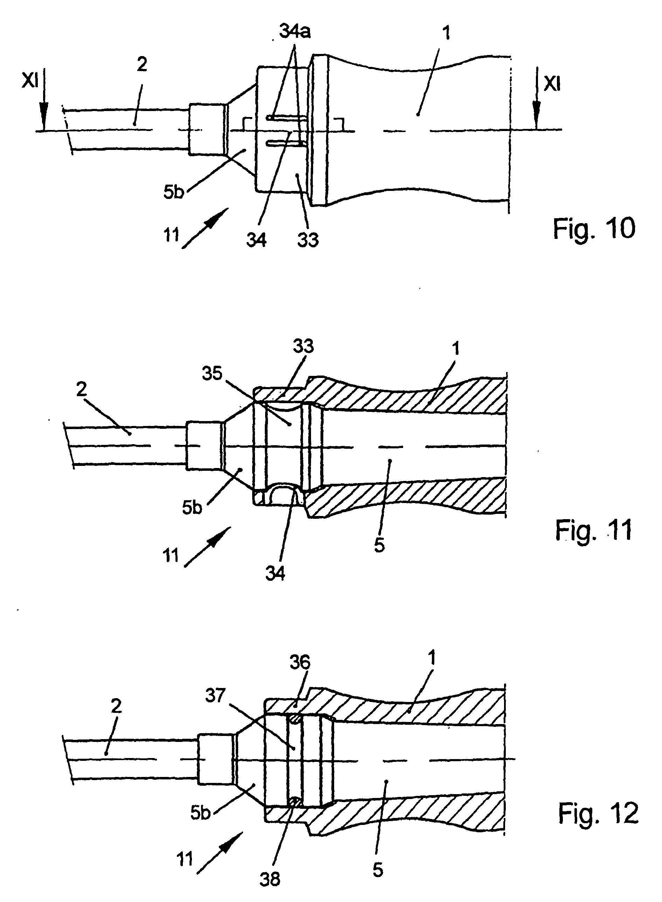

Für eine abweichende Ausgestaltung der Haltevorrichtung gemäß **Fig. 10 und 11** ist eine sattel- oder zungenförmige Feder 34 einstückig in den Ansatz 33 an der Stirnseite des Griffs 1 eingeformt. Der radial innen liegende Bereich der Feder 34 ragt in die Bohrung im Griff 1 hinein, so dass die Feder 34 radial ausgelenkt wird, wenn das Endstück 5 mit dem Kopf 5b in den Griff 1 eingesteckt wird. Durch die Auslenkung wird eine radial wirkende Kraft erzeugt, die das Endstück 5 bzw. das Funktionsteil 2 im Griff 1 hält. Dies kann entweder allein durch die Federkraft, also kraftschlüssig, erfolgen oder zusätzlich formschlüssig, indem in den Kopf 5b eine Ringnut 35 oder eine sonstige Vertiefung eingeformt ist, in die die Feder 34 eingreift. Ist an dem Endstück 5 ein Kopf 5b nicht vorgesehen, so ist in das Endstück 5 selbst eine sattelartige Vertiefung eingeformt, in die die Feder 34 eingreift. Die Draufsicht Fig. 10 auf die Haltevorrichtung 11 dieser Ausführungsvariante zeigt die beiden Schlitze 34a, die in den Ansatz 33 radial eingeformt sind und bis zur Bohrung im Ansatz 33 bzw. der Höhlung 6 im Griff 1 durchgehen. Dadurch wird die Sattelfeder 34 einstückig mit dem Ansatz 33 ausgeformt und kann radial federn.

Abweichend zu den dargestellten Ausführungsformen ist eine kinematische Umkehrung des Prinzips der Haltevorrichtung ebenfalls möglich. Beispielsweise kann eine elastische Nase nicht gegenüber dem Griff abgestützt sein, sondern vielmehr an dem Funktionsteil 2 vorgesehen sein und in eine geeignete Vertiefung des Griffs zur Erzielung der Haltewirkung eintreten.

Für eine weitere Ausführungsform gemäß **Fig. 12** ist in den Kopf 5b des Endstücks 5 eine Ringnut 37 eingeformt, in die ein Ring 37 aus elastischem Material, Gummi oder Kunststoff eingesetzt ist. Der Außendurchmesser des Rings 38 ist so gewählt, dass der Ring 38 an seinem Umfang über die Außenkontur des Kopfes 5b hervorsteht. Der Griff 1 oder der Ansatz 36 an der Vorderseite des Griffs 1 weist eine zylindrische Höhlung auf, deren Innendurchmesser geringfügig größer ist als der Außendurchmesser des Kopfes 5b, aber geringfügig kleiner als der Außendurchmesser des Rings 38, so dass dieser beim Einstecken des Endstücks 5 in den Griff 1 radial zusammengepresst wird und das Funktionsteil 2 durch die entstehenden Reibungskräfte in dem Griff 1 gehalten wird.

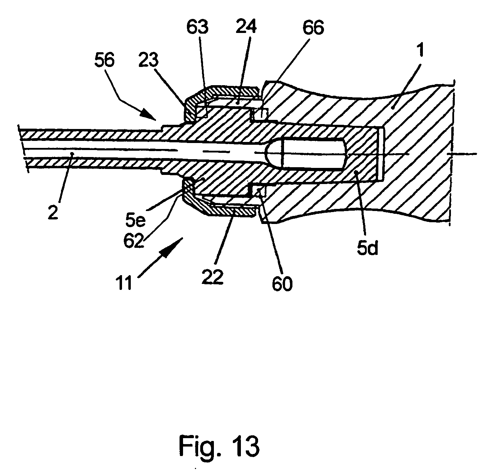

Für eine weitere Ausführungsform gemäß **Fig. 13** besitzt die Höhlung 6 in dem Griff 1 eine kreisringförmige Stufe 66. An der Stirnseite der Stufe 66 ist eine Verzahnung 60 vorgesehen, die in eine entsprechende Verzahnung an der Rückseite des Kopfes 5b des Endstücks 5 eingreift. Die Verzahnung kann axial ausgerichtet sein, wie dies in Fig. 13 dargestellt ist, oder auch radial, wobei der Kopf 5b zweckmäßigerweise tiefer in den Griff 1 eingelassen ist, damit am Griff 1 der größere Durchmesser ausreichende Wanddicke und Festigkeit bietet. Eine Schraubkappe 22 ist auf den Gewindeansatz 24 aufgeschraubt und liegt mit der zweiten Gegenfläche 63 der Stirnwand 23 an der Funktionsfläche 62 des Kopfes 5b an, wodurch das Endstück 5 im Griff 1 gehalten wird. Das Zahnprofil wird zweckmäßigerweise rechteckig gewählt. Das Hinterteil des Endstücks 5 hat vorzugsweise einen unrunden Querschnitt und verläuft konisch.

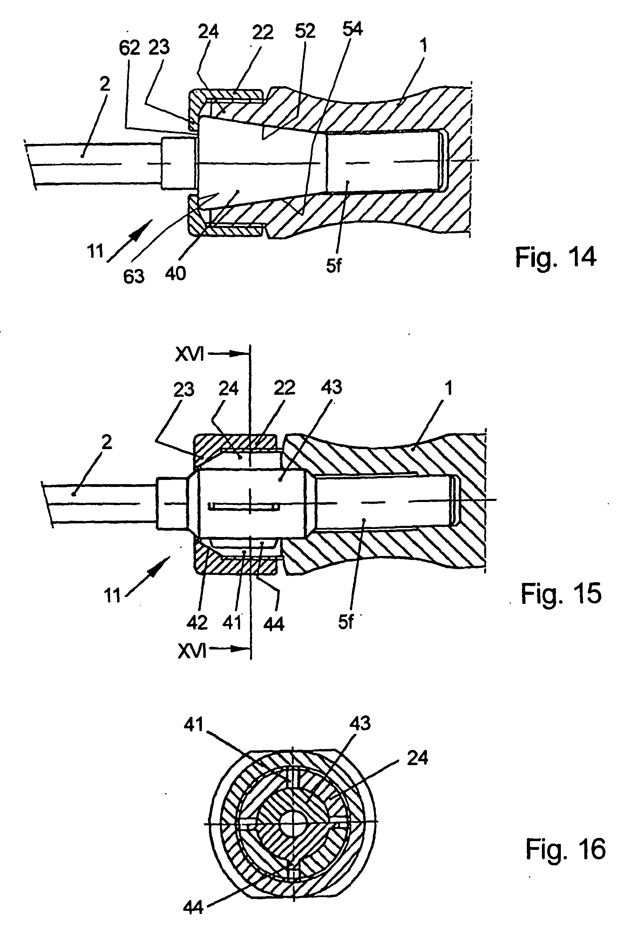

Die zu Fig. 1-13 beschriebenen Formen des Endstücks 5, bzw. der Haltevorrichtungen 11, ergeben eine formschlüssige Verbindung von Griff 1 und Endstück 5, die auch vorzugsweise bei dem Schraubendreher 50 zur Anwendung kommt. Bei den für einen Handschraubendreher in Betracht kommenden Betätigungsmomenten ist bei entsprechender konstruktiver Ausbildung auch eine kraftschlüssige Verbindung ausreichend. Eine solche Verbindung ist in **Fig. 14** mit der Haltevorrichtung 11 dargestellt. Der hintere Bereich des Endstücks 5 ist als zylindrischer Zapfen 5f ausgebildet. Der Zapfen 5f geht in Richtung des Klingenschafts 3 des Funktionsteils 2 in einen Kegel 40 über, der in Richtung der Funktionsspitze geöffnet ist. Eine Schraubkappe 22 auf einem Gewindeansatz 24 an der Stirnseite des Griffs 1 greift mit ihrer Vorderwand 23, die die zweite Gegenfläche 63 bildet, über die Stirnseite des Kegels 40, die die zweite Funktionsfläche 62 bildet, und drückt das Funktionsteil 2 beim Festschrauben in die als Innenkegel ausgeformte Funktionsfläche 54 der Höhlung 6 des Griffs 1, so dass durch Reibung an den Funktionsflächen 54, 62 und Gegenflächen 52, 63 eine kraftschlüssige Verbindung geschaffen ist.

Eine andere kraftschlüssige Verbindung von Griff 1 und Endstück 5 ist ungefähr entsprechend einer Spannzange ausgeführt. Eine derartige Haltevorrichtung 11 ist in **Fig. 15** dargestellt. Bei dieser schließt sich an den zylindrischen Zapfen 5f des hinteren Bereichs des Endstücks 5 in Richtung zum Klingenschaft 3 des Funktionsteils 2 ein zweiter, vorteilhaft im Durchmesser vergrößerter Zylinder 43 an. Der Gewindeansatz 24 ist an der Stirnseite des Griffs 1 mit Schlitzen 41 versehen und an seiner Vorderseite außen konisch verjüngend ausgebildet. Die Schraubkappe 22 weist im vorderen Bereich einen Innenkonus 42 auf, der beim Festschrauben der Schraubkappe 22 auf den Außenkonus des Gewindeansatzes 24 eine Radialkraft ausübt und den Gewindeansatz 24 radial zusammenpresst. Auf diese Weise wird die Innenfläche des Gewindeansatzes 24 auf ein zylindrisches Vorderteil 43 des Endstücks 5 gepresst, so dass eine kraftschlüssige Verbindung zwischen Griff 1 und Endstück 5 entsteht. Zusätzlich können auf dem zylindrischen Vorderteil 43 Rippen 44 vorgesehen sein, die in die Schlitze 41 im Gewindeansatz 24 eingreifen und eine zusätzliche formschlüssige Verbindung ergeben. Dies ist in der unteren Hälfte der Schnitte in Fig. 15 und **Fig. 16** dargestellt.

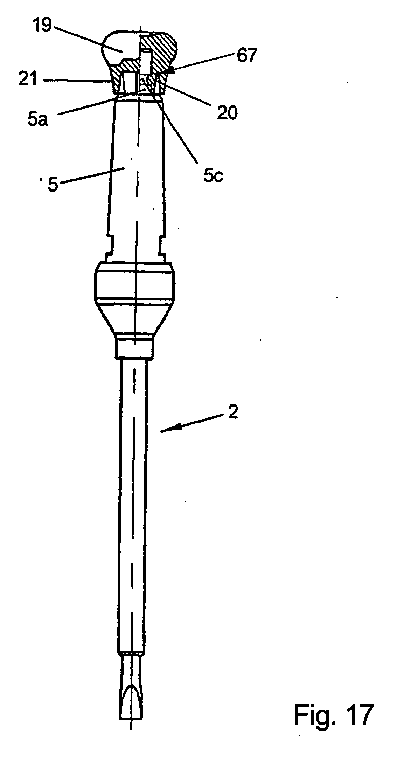

**Fig. 17** zeigt eine weitere Verwendung des Funktionsteils 2: Auf dem Zapfen 5a des Endstücks 5 ist lösbar ein Drehkopf, hier eine Kappe 19, axial fixiert, wobei eine Drehbewegung der Kappe 19 um die Längsachse des Funktionsteils 2 möglich ist. Im Bereich des Zapfens 5a ist eine Ringnut 5c vorgesehen. In diese Ringnut 5c greifen federnde Segmente 20 eines im Übrigen hülsenförmigen, in Längsrichtung geschlitzten Ansatzes 21 der Kappe 19 ein. Die Segmente 20 weisen an ihrer Innenseite in Umfangsrichtung verlaufende Ringnocken auf, welche mit Spiel in die Ringnut 5c eingreifen und die Kappe 19 axial sichern, aber deren leichtgängiges Drehen ermöglichen. Beim Aufstecken und Abziehen der Kappe 19 federn die Segmente 20 radial auf. In axialer Richtung stützt sich die Kappe bei Belastung mit der Innenseite des Bodens der Kappe an der Stirnseite des Zapfens 5a ab. Die Kappe 19 ist leicht drehbar, so dass eine axiale Belastung des Funktionsteils 2 zur Führung des Werkzeugs beim Schrauben nur durch einen Finger aufgebracht werden kann, welcher die Kappe 19 in Längsrichtung beaufschlagt. Der Zapfen 5a kann auch an den Endstücken 5 vorgesehen werden, die in Fig. 7-15 ohne Zapfen dargestellt sind.

Zapfen 5a und Ringnut 5c bilden somit eine weitere Funktionsfläche des Funktionsteils 2, die für ein Zusammenwirken mit der Kappe 19 gestaltet ist und eine lösbare Verbindung des Funktionsteils 2 mit der Kappe 19 ermöglicht. Hierbei ist auch für diese Verbindung eine Isolation zwischen dem Klingenschaft 3 und der Kappe 19 gewährleistet. Dadurch, dass das Funktionsteil 2 einerseits die Funktionsfläche 54 besitzt sowie andererseits die weitere Funktionsfläche 67 für eine Anbindung der Kappe 19, ist ein multifunktionaler Einsatz des Funktionsteils möglich:
a) Einerseits können unterschiedliche Funktionsteile 2 mit einem einzigen Griff 1 betrieben werden.
b) Andererseits kann ohne Einsatz des großen Griffs 1 das Funktionsteil 2 mit der Kappe 19 versehen werden und von dem Benutzer, beispielsweise mit einem Finger, der auf der Kappe 19 liegt, das Funktionsteil 2 auf eine Schraube o. ä. gedrückt werden, wobei mit der anderen Hand das Funktionsteil verdreht werden kann. Eine solche Verwendung ist insbesondere für feinfühliges oder schnelles Schrauben vorteilhaft. Von Vorteil ist weiterhin, dass nicht nur der Griff mit Funktionsteil Isolationsanforderungen wie solche nach EN 60900 erfüllen kann, sondern auch das einzelne Funktionsteil selbst.

Der Grundgedanke der Erfindung ist auch dann verwirklicht, wenn das Endstück 5 in anderer Form als im Beispiel dargestellt ausgebildet ist. In den Figuren ist das Endstück 5 zumindest mit einem runden Kopf 5b dargestellt. Dieser ist zweckmäßig, um zum einen einen guten Abschluss der Höhlung 6 im Griff 1 zu bilden, der etwa kreisförmigen Kontur der Vorderseite des Griffs 1 angepasst, zum anderen eine gute Anlage für Halteelemente wie die Schraubkappe zu bieten. Ein unrundes Querschnittsprofil des Endstücks 5 kann jedoch auch durchgehend ausgeformt sein und an der Übergangsstelle zum Isoliermantel 9 eine Stufe bilden, an die sich die Stirnwand einer Schraubkappe anlegt. Auch können auf der Oberfläche des Endstücks Vertiefungen oder umlaufende Nuten eingeformt sein, in die andere harte Elemente, wie eine Kugel oder ein elastischer Ring, eingreifen bzw. eingesetzt sind. Möglich ist auch, dass eine Abstützung über eine Stufe am Übergang vom Kopf 5b zum hinteren Teil des Endstückes oder mit der hinteren Stirnfläche am Grund 51 der Höhlung 6 im Griff erfolgt. Wesentlich ist, dass das Endstück 5 so ausgebildet ist, dass die Belastungen und Betätigungsmomente übertragen werden können, welche bei sachgemäßer Benutzung der Funktionsteile mit dem Griff in der Praxis auftreten. Auch kann eine Haltevorrichtung 11 oder können deren Elemente anders ausgebildet sein als in den Beispielen dargestellt. Vorzugsweise ist die Haltevorrichtungen vollständig aus Kunststoff hergestellt und das Endstück ist sicher in der Höhlung des Griffs gehalten. Statt einer konischen Ausbildung kann das Endstück 5 auch einen in Längsrichtung konstanten Querschnitt aufweisen.

Die Ausführungsvarianten für die Haltevorrichtung gewährleisten eine sichere Halterung des Funktionsteils im Griff auch dann, wenn ein Funktionsteil auf Zug - in Richtung aus dem Griff heraus - beansprucht wird. Entsprechend dem jeweils vorgesehenen Anwendungsbereich können verschiedene Funktionsteile mit einem Griff zu verschiedenen Satz-Kombinationen zusammengestellt und zweckmäßigerweise in einer Kassette untergebracht werden.

Das Endstück 5 kann von seinem Ende bis zu der der Funktionsspitze 3a zugewandten Stirnseite ein durchgehendes Querschnittsprofil aufweisen oder mit Stufungen, Abschrägungen oder Ausnehmungen ausgebildet sein. Die Wölbung der Außenkontur des Endstücks und der Höhlung 6 im Griff 1 von einer grundsätzlich rechteckförmigen Grundform erzeugt einen relativ großflächigen Kontakt ohne übergroße Kantenpressung. Weitere mögliche Querschnittsprofile sind die eines aus Bogenstrecken gebildeten Sterns mit vorzugsweise drei bis acht Auswölbungen.

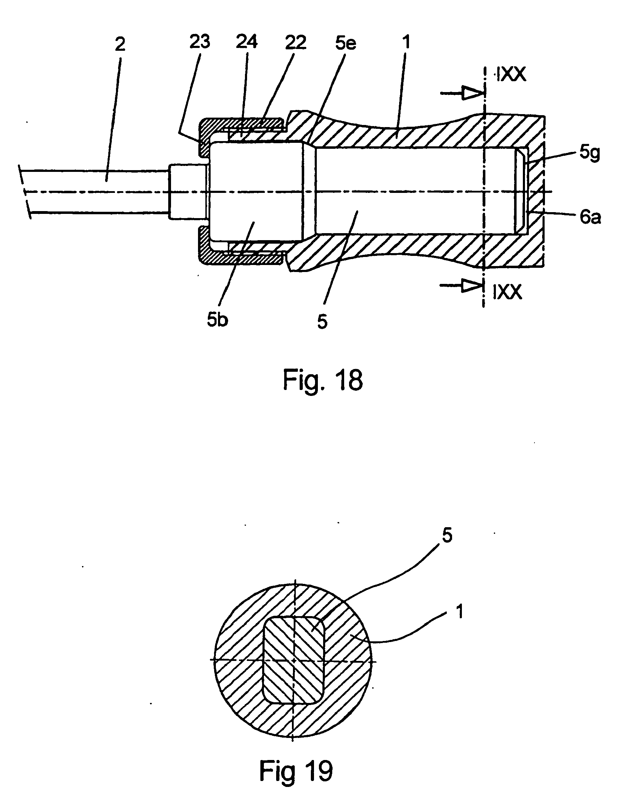

Bei der Ausführungsform gemäß **Fig.18** und **19** hat das Endstück 5 in Längsrichtung keine konische Form, sondern einen gleichbleibenden, vorzugsweise rechteckigen, Querschnitt. Die Höhlung 6 im Griff 1 ist entsprechend geformt. Die Abmessungen und Konturen der Querschnitte des Endstückes 5 und der Höhlung 6 sind so aufeinander abgestimmt, dass ein im Wesentlichen spielfreier Sitz erreicht und ein Betätigungsmoment vom Griff 1 auf das Endstück 5 übertragen werden kann. In Längsrichtung stützt sich das Endstück 5 vorzugsweise an der Stufe 5e ab, die am Übergang vom hinteren Teil des Endstückes 5 in den Kopf 5b ausgebildet ist, und an einer zugeordneten Stufe der Höhlung 6 anliegt. Die außenliegende Stirnfläche des Kopfes 5b bildet eine zweite Funktionsfläche 62, welche an der von der Stirnwand 23 der Schraubkappe 22 gebildeten Gegenfläche 63 zur Anlage kommt. Beim Verschrauben der Schraubkappe 22 mit dem Gewindeansatz 24 wird das Endstück 5 zwischen der Stirnwand 23 und der Stufe 5e verspannt und das Funktionsteil 2 im Griff 1 gehalten. Statt an der Stufe anzuliegen, kann auch vorgesehen werden, dass das Endstück 5 mit seiner Endfläche 5g am Grund 51 der Höhlung 6 anliegt und gegen diesen verspannt wird. Eine Anlage der Stufe 5e an der Stufe in der Höhlung 6 des Griffes 1 ist in diesem Fall nicht vorgesehen.

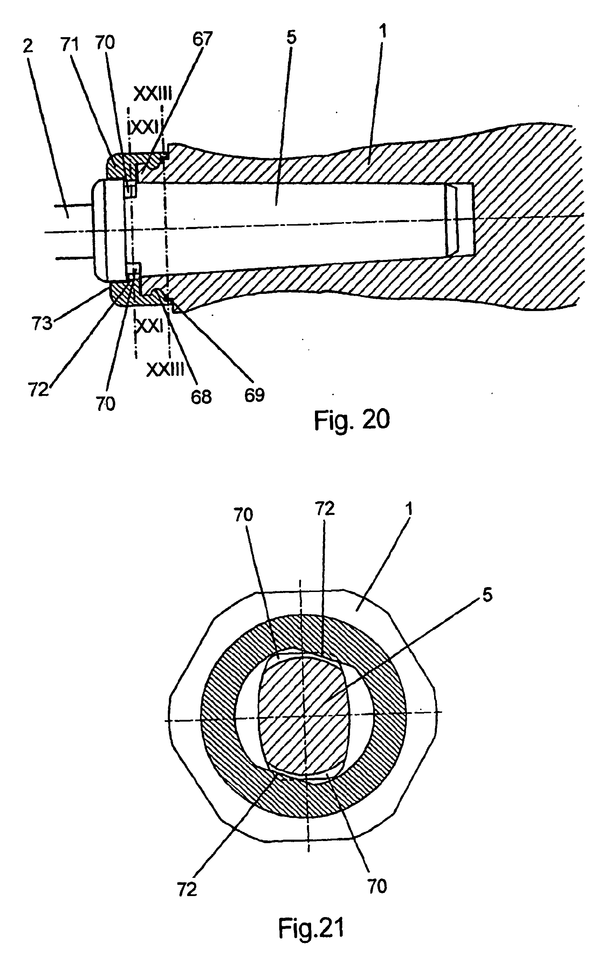

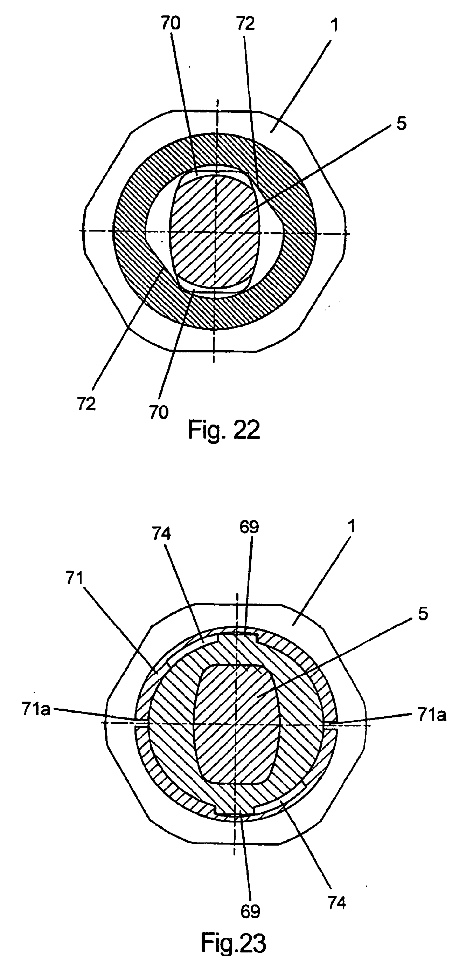

Eine weitere Ausführungsform einer formschlüssig wirkenden Haltevorrichtung ist in Fig. 20 dargestellt. Hierbei weist der Griff 1 an der Vorderseite einstückig einen zylindrischen Ansatz 67 auf, in den eine Ringnut 68 eingeformt ist. Mit einer Schnappverbindung greift in diese Ringnut eine Drehkappe 71 ein, die ausgehend von der Stirnwand 73 eine in Längsrichtung orientierte Durchbrechung aufweist, durch die das Endstück in die Höhlung 6 des Griffes 1 eingesteckt werden kann. In das Endstück 5 sind in seinem vorderen Bereich, bspw. zwei auf dem Umfang gegenüberliegende, Ausnehmungen 70 eingeformt. In diese Ausnehmungen greifen in gesicherter Stellung der Haltevorrichtung zwei nockenartige Segmente 72 ein, die von der zylinderförmigen inneren Mantelfläche der Drehkappe 71 hervorstehen. Zur Sicherung des in den Griff 1 eingesetzten Endstückes 5, also zum Halten des eingesetzten Funktionssteiles 2, wird die Drehkappe 71 für die Ansicht gemäß **Fig. 21** gegen den Uhrzeigersinn gedreht, insbesondere von einer 2-Uhr-Position sowie 8-Uhr-Position der Segmente 72 (gelöste Stellung, **Fig. 22**) in eine 12-Uhr-Position sowie 6-Uhr-Position der Segmente 72 (gesicherte Stellung, Fig. 21). Dadurch kommen die Segmente 72 zum Eingriff in die Ausnehmungen 70 im Endstück 5 und halten dieses axial gesichert im Griff 1. Für eine Umkehrung der vorgenannten Drehung kommen die Segmente 72 außer Eingriff und seitlich vom Endstück 5 zu stehen. In der gelösten Stellung gemäß Fig. 22 kann das Endstück 5 aus dem Griff 1 herausgezogen und gegebenenfalls ein anderes Funktionsteil 2 in den Griff 1 eingesetzt werden. Werden die zur Anlage kommenden Flächen der Ausnehmungen 70 und der Segmente 72 so gestaltet, dass sie in Umfangsrichtung gegenläufig spiralförmig unter einem Winkel verlaufen, so wird beim Verdrehen der Drehkappe 71 gegen den Uhrzeigersinn das Endstück 5 axial in die Höhlung 6 im Griff 1 gedrückt und nicht nur axial gesichert. Beim Drehen der Drehkappe 71 in die andere Richtung werden die Segmente 72, wie zuvor beschrieben, wieder außer Eingriff mit den Ausnehmungen 72 gebracht.

Damit beim Wechseln eines Funktionsteiles 2 die Drehkappe 71 nur um einen möglichst kleinen Winkel gedreht werden muss und die Durchbrechung in der Drehkappe 71 möglichst kongruent zur Öffnung der Höhlung 6 im Griff 1 zu stehen kommt, also die Einstecköffnung beim Einstecken eines Funktionsteiles 2 leicht gefunden werden kann, kann der Drehwinkel durch Anschlagnocken 69 begrenzt werden, die an der Stirnseite des Griffes 1 angeformt sind. Diese Anschlagnocken 69 greifen in entsprechende Ausnehmungen 74 am hinteren Rand der Drehkappe 71 ein. **Fig. 23** zeigt die Eingriffssituation der Einrichtung zur Begrenzung des Drehwinkels. Die Schlitze 71a sind zwei Schlitze im hinteren Teil der Drehkappe 71, die ein Auffedem beim Aufsetzen der Drehkappe auf den zylindrischen Ansatz 67 ermöglichen und Elemente der Schnappverbindung sind.

### BEZUGSZEICHENLISTE

- 1: Griff
- 2: Funktionsteil
- 3: Klingenschaft
- 3a: Funktionsspitze
- 4: Mitnehmerfläche
- 5: Endstück
- 5a: Zapfen
- 5b: Kopf
- 5c: Ringnut
- 5d: Hinterteil
- 5e: Stufe
- 5f: Zapfen
- 5g: Endfläche
- 6: Höhlung
- 7: Druckfeder
- 8: gewölbte Seitenfläche
- 9: Isoliermantel
- 9a: Endbereich Isoliermantel
- 10: Ausnehmung Endstück
- 11: Haltevorrichtung
- 12: Bügel
- 12a: Seitenteil
- 12b: Unterteil / Halteelement
- 12c: Ausnehmung
- 12d: Federbogen, schräg
- 13: Durchbrechung
- 14: Betätigungskopf
- 15: Stütznocken
- 16: Druckfeder
- 17: Federzunge
- 18: Vertiefung
- 19: Kappe
- 20: Segment
- 21: Ansatz
- 22: Schraubkappe
- 23: Stirnwand
- 24: Gewindeansatz
- 25: Schraubkappe
- 26: Innenkonus
- 27: Kugel
- 28: kalottenartige Vertiefung
- 29: Ringnut
- 30: Ringnut
- 31: Ansatz am Griff
- 32: Ringfeder
- 33: Kalotten-Ansatz an Ringfeder
- 34: Ansatz
- 34a: Schlitze
- 35: Feder
- 36: Ringnut
- 37: Ansatz
- 38: Ringnut
- 39: elastischer Ring
- 40: kegelförmiges Vorderteil
- 41: Schlitz
- 42: Konus
- 43: zylindrisches Vorderteil Endstück
- 44: Rippe
- 50: Schraubendreher
- 51: Grund
- 52: Gegenfläche
- 53: Durchbrechung
- 54: Funktionsfläche
- 55: Zapfenbund
- 57: Grund
- 58: Einführschräge
- 59: Bohrung
- 60: Stirnverzahnung
- 61: Bohrung
- 62: zweite Funktionsfläche
- 63: zweite Gegenfläche
- 64: Ringnut
- 65: Durchbrechung
- 66: Stufe
- 67: zylindrischer Ansatz
- 68: Ringnut
- 69: Anschlagnocken
- 70: Ausnehmung
- 71: Drehkappe
- 71a: Schlitz
- 72: Segment
- 73: Stirnwand
- 74: Ausnehmung

## Patentansprüche

1. Schraubendreher mit
- einem Griff (1),
- einem auswechselbaren Funktionsteil (2) mit einem Klingenschaft (3), der teilweise mit einem Isoliermantel (9) aus Kunststoff umhüllt ist und einem isolierenden Endstück (5), welches auswechselbar in den Griff (1) einsetzbar ist und zur Übertragung eines Betätigungstorsionsmomentes zwischen Funktionsteil (2) und Griff (1) mit dem Griff (1) in Wechselwirkung steht,
- einer Haltevorrichtung (11), über die das Funktionsteil (2) in einer gesicherten Stellung der Haltevorrichtung (11) zumindest in eine axiale Richtung gegenüber dem Griff (1) formschlüssig gesichert ist,
- wobei der Griff (1) gegenüber dem Klingenschaft (3) durch Zwischenschaltung des Isoliermantels (9), des Endstückes (5) und/oder der Haltevorrichtung (11) elektrisch isoliert ist, so dass der Schraubendreher für Arbeiten an unter Spannung stehenden Anlagen geeignet ist, und
- die Haltevorrichtung (11) über eine manuelle Betätigung in eine gelöste Stellung der Haltevorrichtung (11) verbracht werden kann, in der das Funktionsteil (2) aus dem Griff (1) herausgezogen werden kann.

2. Schraubendreher nach Anspruch 1, **dadurch gekennzeichnet, dass** die Haltevorrichtung (11) auf die Mantelfläche des Isoliermantels (9) und/oder auf das isolierende Endstück (5) einwirkt.

3. Schraubendreher nach Anspruch 1 oder 2, **dadurch gekennzeichnet, dass** die Haltevorrichtung (11) durch eine manuelle radiale Betätigung eines Betätigungselementes (14) von der gesicherten Stellung in die gelöste Stellung verbracht wird.

4. Schraubendreher nach Anspruch 3, **dadurch gekennzeichnet, dass** ein Federelement (16; 17; 12b; 12d) vorgesehen ist, welches bei manueller Betätigung des Betätigungselements (14) in der gelösten Stellung beaufschlagt ist und ohne manuelle Betätigung des Betätigungselements (14) die Haltevorrichtung (11) in die gesicherte Stellung zurückführt.

5. Schraubendreher nach Anspruch 3 oder 4, **dadurch gekennzeichnet, dass** die Haltevorrichtung (11) ein Halteelement (12b) aufweist, welches mit dem Betätigungselement (14) in Wirkverbindung steht und in der gesicherten Stellung der Haltevorrichtung (11) formschlüssig oder reibschlüssig mit dem Funktionsteil (2), insbesondere dem Endstück (5), zusammenwirkt.

6. Schraubendreher nach einem der Ansprüche 3 bis 5, **dadurch gekennzeichnet, dass** die Haltevorrichtung (11) einen Bügel (12) aufweist,
- dessen Halteelement (12b) mit dem Funktionsteil (2), insbesondere mit dem Endstück (5), in Wirkverbindung steht,
- der mit mindestens einem Seitenteil (12a) um die Mantelfläche des Funktionsteils (2), insbesondere des Endstücks (5) herumgeführt ist und
- der in dem dem Halteelement (12b) radial gegenüberliegenden Bereich mit dem Betätigungselement (14) in Wirkverbindung steht.

7. Schraubendreher nach einem der Ansprüche 4 bis 6, **dadurch gekennzeichnet, dass** während eines Einschiebens des Funktionsteils (2) in den Griff (1) die Haltevorrichtung (11) automatisch in die gelöste Stellung gebracht wird und in der Endstellung des Funktionsteils (2) in dem Griff (1) das Federelement (16; 17; ; 12d) die Bewegung der Haltevorrichtung (11) in die gesicherte Stellung veranlasst.

8. Schraubendreher nach Anspruch 6 oder 7, **dadurch gekennzeichnet, dass** der Bügel (12) und das Betätigungselement (14) einstückig ausgebildet sind.

9. Schraubendreher nach Anspruch 8, **dadurch gekennzeichnet, dass** der Bügel (12) in radial orientierten Nuten des Griffes (1) geführt ist, die von einer Durchbrechung (53) auf der Oberseite des Griffes (1) ausgehen, wobei in der Durchbrechung (53) das Betätigungselement (14) angeordnet ist.

10. Schraubendreher nach einem der Ansprüche 6 bis 9, **dadurch gekennzeichnet, dass** einstückig mit dem Bügel (12) mindestens ein Federelement (16; 17; 12b; 12d) gebildet ist.

11. Schraubendreher nach einem der Ansprüche 6 bis 10, **dadurch gekennzeichnet, dass** das Halteelement(12b) oder der Bügel (12) zwei Stütznocken (15) aufweist, die als Widerlager für Federelemente (16) dienen.

12. Schraubendreher nach Anspruch 11, **dadurch gekennzeichnet, dass** die Stütznocken (15) eine Endposition der radialen Bewegung des Halteelementes (11) vorgeben.

13. Schraubendreher nach einem der vorhergehenden Ansprüche, **dadurch gekennzeichnet, dass** ein Betätigungstorsionsmoment um die Längsachse des Schraubendrehers (50) über eine in Umfangsrichtung formschlüssige Verbindung zwischen dem Griff (1) und dem Endstück (5) übertragen wird.

14. Schraubendreher nach Anspruch 13, **dadurch gekennzeichnet, dass** das Endstück (5) im Bereich der formschlüssigen Verbindung mit dem Griff (1) gegenüber dem Klingenschaft (3) vergrößerte Außenabmessungen aufweist.

15. Schraubendreher nach einem der Ansprüche 6 bis 14, **dadurch gekennzeichnet, dass** der Bügel (12) mit einer Rahmenstruktur gebildet ist, deren Unterteil (12b) das Halteelement bildet, dessen Seitenteile (12a) die Rahmenstruktur in radialer Richtung gegenüber dem Griff (1) führen und dessen Oberteil das Betätigungselement (14) bildet.

16. Schraubendreher nach einem der Ansprüche 4 bis 15, **dadurch gekennzeichnet, dass** zwischen Griff (1) und Funktionsteil (2) ein weiteres Federelement (7) vorgesehen ist, welches
- in Richtung der Längsachse des Schraubendrehers beaufschlagbar ist,
- bei in den Griff (1) eingeschobenem Funktionsteil (2) beaufschlagt ist und
- bei manueller Überführung der Haltevorrichtung (11) in die gelöste Stellung das Funktionsteil (2) zumindest teilweise aus dem Griff (1) ausschiebt oder auswirft.

17. Schraubendreher nach einem der vorhergehenden Ansprüche, **dadurch gekennzeichnet, dass** das Funktionsteil (2) in einer Betriebsstellung und bei Haltevorrichtung (11) in gesicherter Stellung fest mit dem Griff (1) verbunden ist und in einer Entnahmestellung, in der das Funktionsteil (2) teilweise von dem Griff (1) gelöst ist, gegenüber einem Herausfallen gesichert ist, aber von dem Benutzer aus dem Griff (1) entnehmbar ist.

18. Schraubendreher nach einem der vorhergehenden Ansprüche, **dadurch gekennzeichnet, dass** die Form des Endstückes (5) und einer Höhlung (6) des Griffes (1) so aufeinander abgestimmt sind, dass bei Drehmomentbelastung und/oder Axialbelastung ein im Wesentlichen spielfreier Sitz des Endstückes (5) in der Höhlung (6) erzielt wird.

19. Schraubendreher nach einem der vorhergehenden Ansprüche, **dadurch gekennzeichnet, dass** das Funktionsteil (2) als selbständiger Schraubendreher verwendet werden kann, bei welchem der Klingenschaft isoliert ist.

20. Schraubendreher nach einem der vorhergehenden Ansprüche, **dadurch gekennzeichnet, dass** das Endstück (5) in Längsrichtung konisch ausgebildet ist und in einer Längsrichtung an einer konischen Gegenfläche des Griffes (1) abgestützt ist sowie in die andere Längsrichtung durch eine Haltevorrichtung (11) gehalten ist.

21. Schraubendreher nach Anspruch 20, **dadurch gekennzeichnet, dass** die Konuswinkel größer gewählt sind als der Selbsthemmungswinkel der Materialpaarung, die für den Griff (1) und das Endstück (5) gewählt ist.

22. Schraubendreher nach einem der Ansprüche 1, 2, 13, 14, 16 bis 21, **dadurch gekennzeichnet, dass**
- sich das auswechselbare Funktionsteil (2) mit dem Endstück (5) in einer Betriebsstellung mit einer ersten Funktionsfläche (54) in eine axiale Richtung gegenüber einer korrespondierenden ersten Gegenfläche (52) des Griffes (1) abstützt,
- der Griff (1) ein Gewinde aufweist,
- eine Haltevorrichtung (11) mit einer zweiten Gegenfläche (63) und einem Gewinde vorgesehen ist, wobei die zweite Gegenfläche (63) mit einer zweiten Funktionsfläche (62) des Funktionsteiles (2) in Wechselwirkung tritt,
- die Haltevorrichtung (11) und der Griff (1) über eine Verschraubung der Gewinde miteinander verbunden sind und
- über eine Veränderung der Verschraubung eine Veränderung des Abstandes der ersten Gegenfläche (52) von der zweiten Gegenfläche (63) und/oder eine Veränderung der Anpresskraft des Funktionsteiles (2) an die erste Funktionsfläche (54) und die zweite Funktionsfläche (62) herbeiführbar ist.

23. Schraubendreher nach Anspruch 22, **dadurch gekennzeichnet, dass** die Haltevorrichtung (11) im Halbquerschnitt ungefähr L-förmig ausgebildet ist, wobei an einer innenliegenden Seite eines um die Längsachse des Schraubendrehers umlaufenden Schenkels des L das Gewinde der Haltevorrichtung (11) vorgesehen ist und ein quer zur Längsachse des Schraubendrehers orientierter Schenkel des L die zweite Gegenfläche (63) bildet.

24. Schraubendreher nach Anspruch 22, **dadurch gekennzeichnet, dass** die Haltevorrichtung (11) eine konusförmige Innenfläche besitzt, die die zweite Gegenfläche (63) bildet.

25. Schraubendreher nach Anspruch 22, **dadurch gekennzeichnet, dass** die Haltevorrichtung (11) eine konusförmige Innenfläche besitzt, an der eine Kugel (27) anliegt, welche mit zunehmender Verschraubung radial nach innen an eine Vertiefung oder Nut (Ringnut 28) des Funktionsteiles (2) angepresst wird.

26. Schraubendreher nach Anspruch 25, **dadurch gekennzeichnet, dass** die Kugel (27) in dem Griff (1) radial geführt ist.

27. Schraubendreher nach einem der Ansprüche 1, 2, 13, 14, 16 bis 21, **dadurch gekennzeichnet, dass** das Endstück (5) aus einem im wesentlichen konischen Hinterteil (5d) mit runden Querschnitt und einem Vorderteil (5e) besteht, das im Durchmesser größer ist als das Hinterteil (5d), das Vorderteil (5e) mit einer aus mindestens einem Zahn bestehenden radial oder axial ausgerichteten Verzahnung (39) in den Griff (1) eingreift und das Endstück (5d, 5e) durch eine Schraubkappe (22) gehalten wird, die auf einen Gewindeansatz (24) an der Vorderseite des Griffes (1) aufgeschraubt ist.

28. Schraubendreher nach einem der Ansprüche 1, 2, 13, 14, 16 bis 21, **dadurch gekennzeichnet, dass** das Endstück (5) im hinteren Bereich einen zylindrischen Zapfen (5f) aufweist, das Vorderteil als Kegel (40) ausgebildet ist und das Endstück (5) durch eine Schraubkappe (22) in den Griff (1) eingepresst wird, die auf einen Gewindeansatz (24) an der Vorderseite des Griffes (1) aufgeschraubt ist und mit ihrer Stirnwand (23) an der Vorderseite des Kegels (40) anliegt.

29. Schraubendreher nach einem der Ansprüche 1, 2, 13, 14, 16 bis 21, **dadurch gekennzeichnet, dass** ein Gewindeansatz (24) an der Stirnseite des Griffes (1) mit Schlitzen (41) versehen ist und an seiner Vorderseite konisch ausgebildet ist, eine Schraubkappe (22) im vorderen Bereich einen Innenkonus (42) aufweist und bei einem Aufschrauben der Schraubkappe (22) auf den Gewindeansatz (24) der Gewindeansatz (24) radial zusammengedrückt wird, wodurch in der Art eines Spannfutters eine kraftschlüssige Verbindung zwischen Griff (1) und Endstück (5) geschaffen wird.

30. Schraubendreher nach Anspruch 29, **dadurch gekennzeichnet, dass** das Endstück mindestens eine Rippe (44) aufweist, die formschlüssig in Umfangsrichtung in einem Schlitz (41) im Gewindeansatz (24) angeordnet ist.

31. Schraubendreher nach einem der Ansprüche 1, 2, 13, 14, 16 bis 21, **dadurch gekennzeichnet, dass** gegenüber dem Griff (1) verdrehbar um die Längsachse des Schraubendrehers eine Drehkappe (71) abgestützt ist, die mindestens ein radial nach innen hervortretendes Segment (72) aufweist, welches in der gesicherten Stellung formschlüssig in Längsrichtung des Schraubendrehers in einer Ausnehmung (70) des Endstückes (5) angeordnet ist und bei Verdrehung der Drehkappe (71) von der gesicherten Stellung in die gelöste Stellung außer Eingriff mit dem Endstück (5) kommt.

32. Schraubendreher nach Anspruch 31, **dadurch gekennzeichnet, dass** mindestens ein Anschlagnocken (69) an der Drehkappe (71) oder dem Endstück (5) vorgesehen ist, der in einer Ausnehmung (74) von dem anderen Bauteil von dem Endstück (5) oder der Drehkappe (71) angeordnet ist und die Verdrehung der Drehkappe (71) begrenzt.

## Claims

1. Screw driver with
- a handle (1),
- an exchangeable functional part (2) comprising a shank (3), which is at least partially covered by an insulating sheet (9) of plastic, and an insulating end part (5) suitable for an exchangeable introduction into the handle (1) and for an interaction with the handle (1) for transmission of a torsional moment between the functional part (2) and the handle (1),
- a holding device (11) for securing the functional part (2) with respect to the handle (1) by positive engagement in a secured position of the holding device (11) at least in one axial direction,
- wherein the handle (1) is electrically insulated with respect to the shank (3) by means of interposition of the insulating cover (9), the end part (5) and/or the holding device (11) so that the screw driver is suitable for works at devices being subjected to voltage, and
- the holding device is designed for being transferred into an unsecured position by means of a manual activation, wherein in the unsecured position the functional part (2) might be pulled out of the handle (1).

2. Screw driver according to claim 1, wherein the holding device (11) interacts with the outer circumference of the insulating sheet (9) and/or of the insulated end part (5).

3. Screw driver according to claim 1 or 2, wherein the holding device (11) is designed for being transferred from the secured position to the unfastened position by a manual activation of an activation element (14) in radial direction.

4. Screw driver according to claim 3, wherein a spring element (16; 17; 12b; 12d) is provided which is tensed in the unsecured position during manual activation of the activation element (14) and which returns the holding device (11) without manual activation of the actuation element (14) into the secured position.

5. Screw driver according to claim 3 or 4, wherein the holding device (11) comprises a holding element (12b) which interacts with the activation element (14) and cooperates by frictional engagement or positive engagement with the functional part (2), in particular the end part (5).

6. Screw driver according to one of claims 3 to 5, wherein the holding device (11) comprises a bow (12) wherein
- a holding element (12b) of the bow (12) cooperates with the functional part (2), in particular with the end part (5),
- at least one side part (12a) of the bow (12) is extends in circumferential direction of the outer circumference of the functional part (2), in particular the end part (5), and
- the bow cooperates with the activation element (14) in a region wherein said region is located oppositely of the holding element (12b) in radial direction.

7. Screw driver according to one of claims 4 to 6, wherein during introduction of the functional part (2) into the handle (1) the holding device (11) is automatically transferred into the unsecured position and wherein when reaching the end position of the functional part (2) in the handle (1) the spring element (16, 17, 12b, 12d) causes movement of the holding device (11) into the secured position.

8. Screw driver according to claim 6 or 7, wherein the bow (12) and the activation element (14) are built integrally.

9. Screw driver according to claim 8, wherein the bow (12) is guided in grooves of the handle (1) with radial orientation, said grooves extending from an opening (53) at the upper side of the handle (1), wherein the activation element (14) is located in the opening (53).

10. Screw driver according to one of claims 6 to 9, wherein at least one spring element (16, 17, 12b; 12d) is built integrally with the bow (12).

11. Screw driver according to one of claims 6 to 10, wherein the holding element (12b) or bow (12) comprises two supporting protrusions (15) building a support for the spring elements (16).

12. Screw driver according to claim 11, wherein the supporting protrusions (15) provide an end position for the radial movement of the holding element (11).

13. Screw driver according to one of claims 1 to 12, wherein a torsional moment with respect to the longitudinal axis of the screw driver (50) is transmitted by a connection between the handle (1) and the end part (5) said connection comprising a positive engagement in circumferential direction.

14. Screw driver according to claim 13, wherein the end part (5) comprises extended outer dimensions with respect to the shank (3) in the region of the positive engagement with the handle (1).

15. Screw driver according to one of claims 6 to 14, wherein the bow (12) is built with a frame structure, wherein a bottom part (12b) builds the holding element, the side parts (12a) lead the frame structure in radial direction with respect to the handle (1) and the top part builds the activation element (14).

16. Screw driver according to one of claims 4 to 15, wherein between the handle (1) and functional part (2) another spring element (7) is provided which
- might be tensed in longitudinal direction of the screw driver,
- is tensed with an introduction of the functional part (2) into the handle (1) and
- for manual transfer of the holding device (11) into the unsecured position ejects or moves the functional part (2) at least partially out of the handle (1).

17. Screw driver according to one of claims 1 to 16, wherein in an operating position and with the holding device (11) in the secured position the functional part (2) is fixedly connected with the handle (1) and in a removing position with the functional part (2) being partially ejected or moved out of the handle (1) the functional part (2) being secured against falling out of the handle (1) but being removable by the user.

18. Screw driver according to one of claims 1 to 17, wherein the shape of the end piece (5) and a cavity (6) of the handle (1) are correlated such that for an axial thrust and a torsional moment the end piece (5) is seated in the cavity (6) free from play.

19. Screw driver according to of one claims 1 to 18, wherein the functional part (2) is designed for being used separately as a screw driver with an insulated shank.

20. Screw driver according to one of claims 1 to 19, wherein the end part (5) is conically in longitudinal direction and supported in longitudinal direction at a conical counter surface of the handle (1) and supported in the opposite longitudinal direction by the holding device (11).

21. Screw driver according to claim 20, wherein the opening angle of the conus is chosen to be larger than the self-locking angle of the materials of the handle (1) and the end part (5).

22. Screw driver according to one of claims 1, 2, 13, 14, 16 to 21, wherein
- in an operating position the exchangeable functional part (2) is supported with the end part (5) with a first functional surface (54) in one axial direction against a corresponding first counter surface (52) of the handle (1),
- the handle (1) comprises a thread,
- a holding device (11) is provided, the holding device comprising a second counter surface (63) and a thread, wherein the second counter surface (63) interacts with a second functional surface (62) of the functional part (2),
- the holding device (11) and the handle (1) are connected with each other by means of the threads and
- by means of threading the two threads a change of the distance of the first counter surface (52) and the second counter surface (63) and/or a change of the contact force of the functional part (2) at the first functional surface (54) and the second functional surface (62) is caused.

23. Screw driver according to claim 22, wherein the holding device (11) is in semi-cross-section approximately L-shaped, wherein the thread of the holding device (11) being provided at an inner side of a leg of the L, said leg extending in circumferential direction with respect to the longitudinal axis of the screw driver, and wherein the second counter surface (63) is built by another leg of the L, said other leg being oriented transverse to the longitudinal axis of the screw driver.

24. Screw driver according to claim 22, wherein the holding device (11) comprises a conical inner surface building the second counter surface (63).

25. Screw driver according to claim 22, wherein the holding device (11) comprises a conical inner surface being in contact with a ball (27) which is pressed with continued threading radial in inner direction at a recess, cavity or groove (28) of the functional part (2).

26. Screw driver according to claim 25, wherein in the handle (1) the ball (27) is guided in radial direction.

27. Screw driver according to one of claims 1, 2, 13, 14, 16 to 21, wherein the end part (5) is built with the substantially conical back part (5d) with a circulate cross-section and a front part (5e) having a diameter which is larger than the diameter of the back part (5d), wherein the front part (5e) engages the handle with a radial or axial toothing system built with at least one tooth (1) and the end part (5d, 5e) is held by a threaded cap (22) which is threaded with a thread (24) at the front part of the handle (1).

28. Screw driver according to one of claims 1, 2, 13, 14, 16 to 21, wherein the end part (5) comprises a cylindrical extension (5f) at its rear end, the front part forms a cone (40) and the end part (5) is pressed by a threaded cap (22) into the handle (1) which is screwed upon a threaded portion (24) at the front part of the handle (1) and contacts with its front wall (23) the front part of the cone (40).

29. Screw driver according to one of claims 1, 2, 13, 14, 16 to 21, wherein the threaded portion (24) is provided with slots (41) at the front part of the handle (1) and comprises a conical shape at its front part, a threaded cap (22) comprises an inner cone (42) in its front region and by threading the threaded cap (22) upon the threaded region (24) the threaded region (24) is radially compressed for building a non-positive engagement between the handle (1) and the end part (5) which is similar to a chuck.

30. Screw driver according to claim 29, wherein the end part comprises at least one rib (44) which is in positive engagement in circumferential direction with a slot (41) in the threaded region (24).

31. Screw driver according to one of claims 1, 2, 13, 14, 16 to 21, wherein a rotational cap (71) is supported with respect to the handle (1) with a rotational degree of freedom with respect to the longitudinal axis of the screw driver, wherein the rotational cap (71) comprises at least one segment (72) that protrudes in radial inner direction and that in the secured position is in positive engagement in longitudinal direction of the screw driver located in a recess (70) of the end part (5) and wherein the positive engagement with the end part (5) is removed by means of rotating the rotational cap (71) from the secured position into the unsecured position.

32. Screw driver according to claim 31, wherein the rotational cap (71) or the end part (5) comprises at least one abutting protrusion which is located in a recess (74) of the other element of the end part (5) or the rotational cap (71) and limits the rotational degree of freedom of the rotational cap (71).

## Revendications

1. Tournevis comportant
- un manche (1),
- un élément fonctionnel (2) amovible, avec une tige à lame (3), qui est partiellement enveloppée d'une gaine isolante (9) en matière plastique, et une queue (5) isolante, qui peut être montée de manière amovible dans le manche (1) et est en interaction avec le manche (1) pour transmettre un couple de torsion de manoeuvre entre l'élément fonctionnel (2) et le manche (1),
- un dispositif de retenue (11), par lequel l'élément fonctionnel (2), dans une position verrouillée du dispositif de retenue (11), est bloqué par conjugaison de forme au moins dans une direction axiale par rapport au manche (1),
- le manche (1) étant isolé électriquement par rapport à la tige à lame (3) par l'intermédiaire de la gaine isolante (9), de la queue (5) et/ou du dispositif de retenue (11), de telle sorte que le tournevis est adapté à des travaux sur des installations sous tension, et
- le dispositif de retenue (11) peut être amené par l'intermédiaire d'une manoeuvre manuelle dans une position déverrouillée du dispositif de retenue (11), dans laquelle l'élément fonctionnel (2) peut être tiré hors du manche (1).

2. Tournevis selon la revendication 1, **caractérisé en ce que** le dispositif de retenue (11) agit sur la face latérale de la gaine isolante (9) et/ou sur la queue isolante (5).

3. Tournevis selon la revendication 1 ou 2, **caractérisé en ce que** le dispositif de retenue (11) est amené de la position verrouillée dans la position déverrouillée par une manoeuvre radiale manuelle d'un élément d'actionnement (14).

4. Tournevis selon la revendication 3, **caractérisé en ce qu'**il est prévu un élément ressort (16 ; 17 ; 12b ; 12d) qui, lors d'une manoeuvre manuelle de l'élément d'actionnement (14), est sollicité dans la position déverrouillée et, sans manoeuvre manuelle de l'élément d'actionnement (14), ramène le dispositif de retenue (11) dans la position verrouillée.

5. Tournevis selon la revendication 3 ou 4, **caractérisé en ce que** le dispositif de retenue (11) comporte un élément de retenue (12b) qui est en liaison active avec l'élément d'actionnement (14) et, dans la position verrouillée du dispositif de retenue (11), coopère par conjugaison de forme ou par frottement avec l'élément fonctionnel (2), en particulier avec la queue (5).

6. Tournevis selon l'une quelconque des revendications 3 à 5, **caractérisé en ce que** le dispositif de retenue (11) comporte un étrier (12),
- dont l'élément de retenue (12b) est en liaison active avec l'élément fonctionnel (2), en particulier avec la queue (5),
- qui, avec au moins une partie latérale (12a), enserre la face latérale de l'élément fonctionnel (2), en particulier de la queue (5), et
- qui, dans la zone radialement en face de l'élément de retenue (12b), est en liaison active avec l'élément d'actionnement (14).

7. Tournevis selon l'une quelconque des revendications 4 à 6, **caractérisé en ce que**, pendant une introduction de l'élément fonctionnel (2) dans le manche (1), le dispositif de retenue (11) est amené automatiquement dans la position déverrouillée et, dans la position finale de l'élément fonctionnel (2) dans le manche (1), l'élément de ressort (16 ; 17 ; 12d) induit le mouvement du dispositif de retenue (11) dans la position verrouillée.

8. Tournevis selon la revendication 6 ou 7, **caractérisé en ce que** l'étrier (12) et l'élément d'actionnement (14) sont réalisés d'un seul tenant.

9. Tournevis selon la revendication 8 **caractérisé en ce que** l'étrier (12) est guidé dans des rainures du manche (1), qui sont orientées dans le sens radial et qui partent d'une lumière (53) sur le côté supérieur du manche (1), l'élément d'actionnement (14) étant disposé dans la lumière (53).

10. Tournevis selon l'une quelconque des revendications 6 à 9, **caractérisé en ce qu'**au moins un élément ressort (16 ; 17 ; 12b ; 12d) est formé d'un seul tenant avec l'étrier (12).

11. Tournevis selon l'une quelconque des revendications 6 à 10, **caractérisé en ce que** l'élément de retenue (12b) ou l'étrier (12) comportent deux ergots d'appui (15), qui font fonction de contre-butée pour des éléments ressorts (16).

12. Tournevis selon la revendication 11, **caractérisé en ce que** les ergots d'appui (15) définissent une position finale du mouvement radial de l'élément de retenue (11).

13. Tournevis selon l'une quelconque des revendications précédentes, **caractérisé en ce qu'**un couple de torsion de manoeuvre autour de l'axe longitudinal du tournevis (50) est transmis par l'intermédiaire d'un assemblage par conjugaison de forme dans le sens périphérique entre le manche (1) et la queue (5).

14. Tournevis selon la revendication 13, **caractérisé en ce que** la queue (5), dans la zone de l'assemblage par conjugaison de forme avec le manche (1), présente des dimensions extérieures supérieures à celles de la tige à lame (3).

15. Tournevis selon l'une quelconque des revendications 6 à 14, **caractérisé en ce que** l'étrier (12) est réalisé avec une structure de cadre, dont la partie inférieure (12b) forme l'élément de retenue, dont les parties latérales (12a) guident la structure de cadre dans le sens radial par rapport au manche (1) et dont la partie supérieure forme l'élément d'actionnement (14).

16. Tournevis selon l'une quelconque des revendications 4 à 15, **caractérisé en ce qu'**il est prévu, entre le manche (1) et l'élément fonctionnel (2), un élément ressort (7) supplémentaire, lequel
- peut être sollicité dans le sens de l'axe longitudinal du tournevis,
- est sollicité en présence de l'élément fonctionnel (2) introduit dans le manche (1), et
- pousse vers l'extérieur ou éjecte l'élément fonctionnel (2) au moins partiellement hors du manche (1) lorsque le dispositif de retenue (11) est amené manuellement dans la position déverrouillée.

17. Tournevis selon l'une quelconque des revendications précédentes, **caractérisé en ce que** l'élément fonctionnel (2), dans la position de service et lorsque le dispositif de retenue (11) est en position verrouillée, est relié ferment au manche (1) et dans une position de désolidarisation, dans laquelle l'élément fonctionnel (2) est partiellement détaché du manche (1), est bloqué pour ne pas tomber hors du manche (1), mais peut être retiré par l'utilisateur hors de ce dernier.

18. Tournevis selon l'une quelconque des revendications précédentes, **caractérisé en ce que** la forme de la queue (5) et la forme d'une cavité (6) du manche (1) sont adaptées l'une à l'autre de telle sorte que, lors d'une sollicitation par un couple de rotation et/ou d'une sollicitation axiale, la queue (5) est positionnée sensiblement sans jeu à l'intérieur de la cavité (6).

19. Tournevis selon l'une quelconque des revendications précédentes, **caractérisé en ce que** l'élément fonctionnel (2) peut être utilisé sous forme de tournevis autonome, dans lequel la tige à lame est isolée.

20. Tournevis selon l'une quelconque des revendications précédentes, **caractérisé en ce que** la queue (5) est conique dans le sens longitudinal et, dans une direction longitudinale, est en appui sur une surface complémentaire conique du manche (1) et, dans l'autre direction longitudinale, est maintenue par un dispositif de retenue (11).

21. Tournevis selon la revendication 20, **caractérisé en ce que** les angles de cône sont choisis plus grands que l'angle d'autoblocage de l'appariement de matière qui est choisi pour le manche (1) et la queue (5).

22. Tournevis selon l'une quelconque des revendications 1, 2, 13, 14, 16 à 21, **caractérisé en ce que**
- dans une position de service, l'élément fonctionnel (2) amovible avec la queue (5) prend appui avec une première face fonctionnelle (54) dans une direction axiale contre une première face complémentaire (52) correspondante du manche (1),
- le manche (1) comporte un filetage,
- il est prévu un dispositif de retenue (11) avec une deuxième face complémentaire (63) et un filetage, la deuxième face complémentaire (63) entrant en interaction avec une deuxième face fonctionnelle (62) de l'élément fonctionnel (2),
- le dispositif de retenue (11) et le manche (1) étant assemblés l'un à l'autre par un vissage des filetages, et
- une variation du vissage pouvant entraîner une variation de la distance entre la première face complémentaire (52) et la deuxième face complémentaire (63) et/ou une variation de la force de pression de l'élément fonctionnel (2) sur la première face fonctionnelle (54) et la deuxième face fonctionnelle (62).

23. Tournevis selon la revendication 22, **caractérisé en ce que** le dispositif de retenue (11), dans la demi-section transversale, est réalisée sensiblement en forme de L, le filetage du dispositif de retenue (11) étant prévu sur une face intérieure d'une branche du L tournant autour de l'axe longitudinal du tournevis et la deuxième face complémentaire (63) étant formée par une branche du L orientée perpendiculairement à l'axe longitudinal du tournevis.

24. Tournevis selon la revendication 22, **caractérisé en ce que** le dispositif de retenue (11) comporte une face intérieure conique, qui forme la deuxième face complémentaire (63).

25. Tournevis selon la revendication 22, **caractérisé en ce que** le dispositif de retenue (11) comporte une face intérieure conique contre laquelle vient en appui une bille (27) qui, sous l'effet du vissage progressif, est pressée dans le sens radial vers l'intérieur contre un creux ou rainure (rainure annulaire 28) de l'élément fonctionnel (2).

26. Tournevis selon la revendication 25, **caractérisé en ce que** la bille (27) est guidée radialement dans le manche (1).

27. Tournevis selon l'une quelconque des revendications 1, 2, 13, 14, 16 à 21, **caractérisé en ce que** la queue (5) est formée par une partie arrière (5d) sensiblement conique à section ronde et une partie avant (5e), dont le diamètre est plus grand que celui de la partie arrière (5d), la partie avant (5e) s'engage dans le manche (1) avec une denture (39) comportant au moins une dent et orientée dans le sens radial ou axial, et la queue (5d, 5e) est maintenue par un capuchon fileté (22), qui est vissé sur une saillie filetée (24) sur la face avant du manche (1).

28. Tournevis selon l'une quelconque des revendications 1, 2, 13, 14, 16 à 21, **caractérisé en ce que** la queue (5), dans une zone arrière, comporte un tenon (5f) cylindrique, la partie avant est réalisée sous forme de cône (40) et la queue (5) est pressée dans le manche (1) par un capuchon fileté (22), qui est vissé sur une saillie filetée (24) sur la face avant du manche (1) et est en appui avec sa paroi frontale (23) sur la face avant du cône (40).

29. Tournevis selon l'une quelconque des revendications 1, 2, 13, 14, 16 à 21, **caractérisé en ce qu'**une saillie filetée (24) sur la face frontale du manche (1) comporte des fentes (41) et est conique sur sa face avant, un capuchon fileté (22) comporte un cône intérieur (42) dans la zone avant et, lors d'un vissage du capuchon fileté (22) sur la saillie filetée (24), comprime la saillie filetée (24) dans le sens radial, moyennant quoi un assemblage par conjugaison de force à la manière d'un mandrin de serrage est obtenu entre le manche (1) et la queue (5).

30. Tournevis selon la revendication 29, **caractérisé en ce que** la queue comporte au moins une nervure (44) qui est disposée par conjugaison de forme dans le sens périphérique dans une fente (41) dans la saillie filetée (24).

31. Tournevis selon l'une quelconque des revendications 1, 2, 13, 14, 16 à 21, **caractérisé en ce qu'**un capuchon rotatif (71) est en appui par rapport au manche (1) de manière rotative autour de l'axe longitudinal du tournevis, lequel capuchon rotatif comporte au moins un segment (72) en saillie radiale vers l'intérieur qui, dans la position verrouillée par conjugaison de forme dans le sens longitudinal du tournevis, est situé dans un évidement (70) de la queue (5) et, lors de la rotation du capuchon rotatif (71) de la position verrouillée dans la position déverrouillée, est amené hors de prise avec la queue (5).

32. Tournevis selon la revendication 31, **caractérisé en ce que**, sur le capuchon rotatif (71) ou sur la queue (5), il est prévu au moins un téton de butée (69) qui est disposé dans un évidement (74) de l'autre pièce de la queue (5) ou du capuchon rotatif (71) et limite la rotation du capuchon rotatif (71).
